# FASCICULE DE BREVET EUROPEEN

(11) **EP 0 638 572 B1**
(45) Date de publication et mention de la délivrance du brevet: **04.12.2002**
(21) Numéro de dépôt: 94401805.0
(22) Date de dépôt: 04.08.1994
(51) Int. Cl.: C07D 495/04, C07C 311/16

(54) **Nouveaux dérivés bicycliques de l'imidazole, leur procédé de préparation, les nouveaux intermédiaires obtenus, leur application à titre de médicaments et les compositions pharmaceutiques les renfermant**
Bicyclische Imidazolverbindungen, Verfahren zu ihrer Herstellung, Zwischenverbindungen, ihre Anwendung als Medikamente und sie enthaltende pharmazeutische Zusammensetzungen
Bicyclic derivatives of imidazole, process for their preparation, intermediates; their application as medicaments and pharmaceutical compostions containing them

(30) Priorité: 05.08.1993 FR 9309654
(43) Date de publication de la demande: 15.02.1995
(73) Titulaire: Aventis Pharma S.A., 92160 Antony (FR)
(72) Inventeur: Corbier, Alain, F-91370 Verrieres Le Buisson (FR); Vevert, Jean-Paul, F-93500 Pantin (FR); Zhang, Jidong, F-75013 Paris (FR)

(56) Documents cités:
- EP-A- 0 461 040
- EP-A- 0 503 162
- EP-A- 0 533 058

## Description

La présente invention concerne de nouveaux dérivés bicycliques de l'imidazole, leur procédé de préparation, les nouveaux intermédiaires obtenus, leur application à titre de médicaments et les compositions pharmaceutiques les renfermant.

Le document EP 0461040 décrit des dérivés de l'imidazole ayant des propriétés antagonistes pour le récepteur de l'angiotensine II. Le document EP 0503162 décrit des dérivés d'azoles ayant des propriétés antagonistes pour le récepteur de l'angiotensine II. Le document EP 0533058 décrit des dérivés de l'imidazole ayant des propriétés antagonistes pour le récepteur de l'angiotensine II.

La présente invention a pour objet les produits de formule (I) : 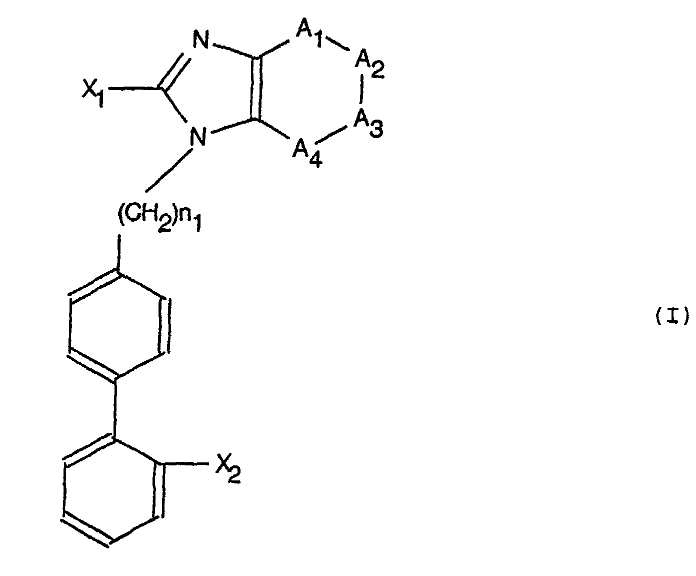 dans laquelle :
X₁ représente un radical méthyle, éthyle, propyle, n-butyle, i-butyle ou terbutyle,
A₁ représente -S-, A₂ représente -CH₂,
A₃ représente -CH₂- ou -CH=, un atome d'hydrogène étant éventuellement substitué par un atome d'halogène, un radical formyle ou carboxy libre, salifié ou estérifié,
A₄ représente >C=O, >C=N-O-CH₂-COOH, >C=N-OH, >C=CH-COOH, 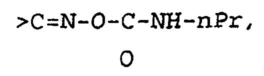 -CH₂- avec l'un ou les 2 atomes d'hydrogène substitués par un ou deux radicaux identiques ou différents choisis parmi les radicaux amino, hydroxyle et halogène, ou - CH= éventuellement substitué par un radical amino, hydroxyle ou un atome d'halogène
X₂ représente le radical tétrazolyle éventuellement salifié ou estérifié, le radical -SO₂-NH-CO-O-R₅, -SO₂-NH-CO-R₅, -SO₂-N=CH-NR₆, ou -SO₂-NH-CO-NH-R₅ dans lesquels R₅ et R₆ identiques ou différents, sont choisis parmi l'atome d'hydrogène, le radical méthyle, éthyle et n-propyle,
lesdits produits de formule (I) étant sous toutes les formes isomères possibles racémiques, énantiomères et diastéréoisomères, ainsi que les sels d'addition avec les acides minéraux et organiques ou avec les bases minérales et organiques, desdits produits de formule (I).

Dans les produits de formule (I) et dans ce qui suit :
- l'atome de soufre peut être oxydé sous forme de sulfoxyde ou de sulfone,
- le terme radical alkyle linéaire ou ramifié désigne de préférence les radicaux méthyle, éthyle, n-propyle, isopropyle, n-butyle, isobutyle, sec-butyle et tert-butyle mais peut également représenter un radical pentyle ou hexyle et particulièrement isopentyle et isohexyle,
- le terme atome d'halogène désigne de préférence l'atome de chlore, mais peut aussi représenter un atome de fluor, de brome ou d'iode,
- le terme radical acyle ayant de 2 à 6 atomes de carbone désigne de préférence un radical acétyle, propionyle, butyryle ou benzoyle, mais également un radical valéryle, hexanoyle, acryloyle, crotonoyle ou carbamoyle,
- le terme radical acylamino désigne de préférence les groupements renfermant un des radicaux acyle tels que définis ci-dessus et liés à un atome d'oxygène tel que par exemple acétylamino, propionylamino, butyrylamino, valérylamino, carbamoylamino, benzoylamino,
- le terme carboxy estérifié désigne de préférence un groupe alkoxy inférieur carbonyle tel que méthoxycarbonyle, éthoxycarbonyle, tert-butoxycarbonyle ou benzyloxycarbonyle,
- le terme radical alkoxy linéaire ou ramifié désigne de préférence les radicaux méthoxy ou éthoxy, mais peut aussi représenter un radical propoxy, isopropoxy, butoxy linéaire, secondaire ou tertiaire,
- le terme radical alkylthio linéaire ou ramifié désigne les radicaux dans lesquels le radical alkyle peut représenter, par exemple, les valeurs indiquées ci-dessus pour le radical alkyle ; le radical alkylthio représente de préférence les radicaux méthylthio ou éthylthio, mais peut aussi représenter un radical propylthio, isopropylthio, n-butylthio, sec-butyl-thio, tert-butylthio, isopentylthio ou isohexylthio,

Les radicaux alkylpipéridyle et alkylthiazolyle désignent des radicaux dans lesquels les radicaux alkyle peuvent prendre les valeurs définies ci-dessus pour ces radicaux ; comme exemples de tels radicaux on peut citer les radicaux méthylpipéridyle, diméthylpipéridyle, trifluorométhylpipéridyle, méthylthiazolyle, étant entendu que dans la liste non exhaustive d'exemples de radicaux telle que citée ci-dessus, le radical méthyle peut être remplacé tout aussi également par les radicaux éthyle, propyle ou butyle ;
on peut citer encore les radicaux phénylvinyle ou phénylallyle, étant entendu que dans ces radicaux le radical phényle peut être remplacé tout aussi également par le radical pyridyle ou tétrazolyle.
- le terme carbamoyle désigne le radical carbamoyle non substitué ou le radical carbamoyle substitué par exemple un groupe N-monoalkyke inférieur carbamoyle, tel que N-méthylcarbamoyle, N-éthylcarbamoyle, un groupe N,N-dialkyle inférieur carbamoyle, tel que N,N-diméthylcarbamoyle, N,N-diéthylcarbamoyle ; un groupe N-(hydroxyalkyle inférieur) carbamoyle, tel que N-(hydroxyméthyl) carbamoyle, N-(hydroxyéthyl) carbamoyle, un groupe carbamoylalkyle inférieur, tel que carbamoylméthyle, carbamoyléthyle.

Les radicaux amino que peuvent représenter ou porter l'un ou plusieurs des radicaux définis ci-dessus et dans ce qui suit désignent des radicaux dans lesquels à l'atome d'azote sont liés deux radicaux, identiques ou différents, choisis parmi l'atome d'hydrogène ; les radicaux alkyle tels que définis ci-dessus pour donner de préférence les radicaux monoalkyle- ou dialkylamino dans lesquels les radicaux alkyles linéaires ou ramifiés renferment de 1 à 6 atomes de carbone et en particulier des radicaux méthyle, éthyle, isopropyle, trifluorométhyle, pentafluoroéthyle, hydroxyméthyle, hydroxyéthyle, méthoxyméthyle, méthoxyéthyle, éthoxyéthyle ; les radicaux alkényle tels que définis ci-dessus et représentés de préférence par les radicaux vinyle et allyle ; les radicaux phényle, tétrazolyle, benzyle, phénéthyle, naphtyle, indolyle, indolinyle, thiényle, furyle, pyrrolyle, pyridyle, pyrrolidinyle, pipéridino, morpholino, pipérazinyle, ces radicaux pouvant être substitués par un ou plusieurs radicaux comme par exemple dans méthylpipérazinyle, fluorométhylpipérazinyle, éthylpipérazinyle, propylpipérazinyle, phénylpipérazinyle ou benzylpipérazinyle.

Les radicaux ci-dessus représentent par exemple et de façon non exhaustive, les radicaux -NH-aryle tel que -NH-phényle ou -NH-tétrazolyle ; -NH-alkyle ; -N-(alkyle)₂ ; -NH-CO-NH-alkyle tel que -NH-CO-NH-tBu ; -NH-CO-NH-n-propyle; NH-CO-NH-aryle tel que -NH-CO-NH-tétrazolyle ou -NH-CO-NH-pyridyle ou -N(alkyl)-CO-NH-tétrazolyle, étant entendu que dans tous ces radicaux, les radicaux alkyle peuvent prendre les valeurs indiquées ci-dessus pour ces radicaux et être éventuellement substitués ainsi qu'il est indiqué ci-dessus pour ces radicaux.

Lorsque les radicaux liés à l'atome d'azote forment ensemble avec l'atome d'azote auquel ils sont liés un hétérocycle, il s'agit, par exemple, d'un cycle pyrrolyle, imidazolyle, pyridyle, pyrazinyle, pyrimidyle, indolyle, indolinyle, purinyle, quinolyle, pyrrolidinyle, pipéridyle, pipéridino, morpholino, pipérazinyle ; ces radicaux peuvent être éventuellement substitués par les substituants déjà mentionnés précédemment et en particulier par un ou plusieurs radicaux choisis parmi les atomes de chlore et de fluor, les radicaux méthyle, éthyle, isopropyle, tert-butyle, méthoxy, éthoxy, propoxy, benzoyle, méthoxycarbonyle, éthoxycarbonyle, comme par exemple dans méthylpipérazinyle, éthylpipérazinyle, propylpipérazinyle, phénylpipérazinyle ou benzylpipérazinyle : dans ces deux derniers radicaux, les radicaux phényle et benzyle peuvent être substitués comme indiqué précédemment.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique,l'acide alpha, bêta-éthanedisulfonique, les acides arylmonosulfoniques tels que l'acide benzènesulfonique et les acides aryldisulfoniques.

Le ou les radicaux carboxy des produits de formule (I) peuvent être salifiés par des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaine, la lysine, l'arginine, l'histidine, la N-méthylglucamine.

Parmi les produits objet de l'invention, peuvent être cités tout particulièrement :
le produit suivant
acide 2-butyl 1-[[2'-[[(éthoxycarbonyl] amino] sulfonyl] (1,1'-biphényl)-4-yl] méthyl] 1,5,6,7-tétrahydrothiopyrano
[2,3-d] imidazol 7-carboxylique, ou avec les bases minérales et organiques, et également
   - le 4'-[(2-butyl-7-oxo-1,5,6,7-tétrahydro-thiopyrano[2,3-d] imidazol-1-yl) méthyl] N- [(diméthylamino) méthylène) (1,1'-biphényl) 2-sulfonamide,
   - le 4'-[(2-butyl-7-oxo-1,5,6,7-tétrahydro-thiopyrano[2,3-d] imidazol-1-yl) méthyl] (1,1'-biphényl) 2-sulfonamide,
   - le (2-(4'-((2-butyl-1,5,6,7-tétrahydro-7-oxo-thiopyrano[2,3-d] imidazol-1-yl) méthyl) (1,1'-biphényl) sulfonyl) carbamate d'éthyle,
   - le N-(2-(4'-((2-butyl-1,5,6,7-tétrahydro-7-oxo-thiopyrano[2,3-d] imidazol-1-yl) méthyl) (1,1'-biphényl) sulfonyl) N'-propylurée,
   - le 4'-[(2-butyl-7-(hydroxyimino)-1,5,6,7-tétrahydro-thiopyrano[2,3-d] imidazol-1-yl) méthyl] (1,1'-biphényl) 2-sulfonamide,
   - le 4'-[(2-butyl-7-((((propylamino) carbonyl) oxy) imino)-1,5,6,7-tétrahydro-thiopyrano[2,3-d] imidazol-1-yl) méthyl] N- [(propylamino) carbonyl] (1,1'-biphényl) 2-sulfonamide,
   - 4'-[(2-butyl 7-(hydroxyimino) 1,5,6,7-tétrahydro thiopyrano [2,3-d]imidazol-1-yl) méthyl] N-((propylamino) carbonyl) (1,1'-biphényl) 2-sulfonamide,
   - le 4'-[(2-butyl-1,7-dihydro-7-oxo-thiopyrano[2,3-d]imidazol-1-yl) méthyl] N-[(diméthylamino) méthylène) (1,1'-biphényl) 2-sulfonamide,
   - le 4'-[(2-butyl-1,7-dihydro-7-oxo-thiopyrano[2,3-d]imidazol-1-yl) méthyl] (1,1'-biphényl) 2-sulfonamide,
   - le 4'-[(2-butyl-1,7-dihydro-7-oxo-thiopyrano[2,3-d]imidazol-1-yl) méthyl] N-[(propylamino) carbonyl) (1,1'-biphényl) 2-sulfonamide, lesdits produits de formule (I) ce produit étant sous toutes les formes isomères possibles racémiques, énantiomères et diastéréoisomères, ainsi que les sels d'addition avec les acides minéraux et organiques.

L'invention a également pour objet un procédé de préparation des produits de formule (I) telle que définie ci-dessus, caractérisé en ce que l'on fait réagir un composé de formule (II) : 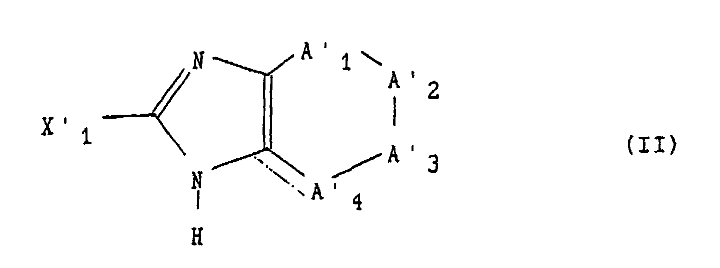 dans laquelle
X'₁, A'₁, A'₂, A'₃ et A'₄ ont les significations indiquées ci-dessus respectivement pour X₁,
A₁, A₂, A₃ et A₄, dans lesquelles les éventuelles fonctions réactives sont éventuellement protégées par des groupements protecteurs,
avec un composé de formule (III) : 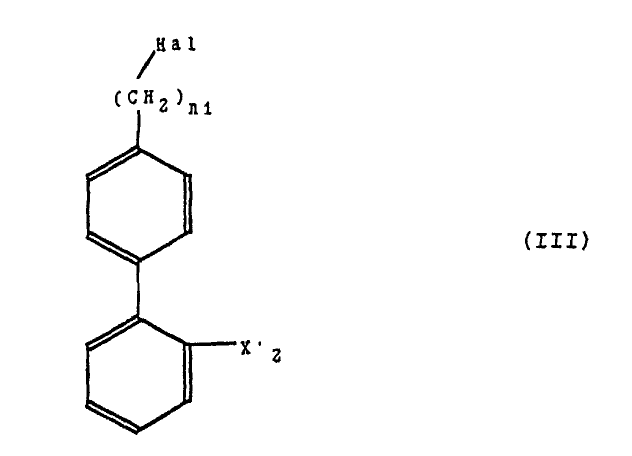 dans laquelle Hal représente un atome d'halogène, n₁ représente un entier de 0 à 4 et X'₂ a la signification indiquée ci-dessus pour X₂ dans laquelle les éventuelles fonctions réactives sont éventuellement protégées par des groupements protecteurs, pour obtenir un produit de formule (IV) : 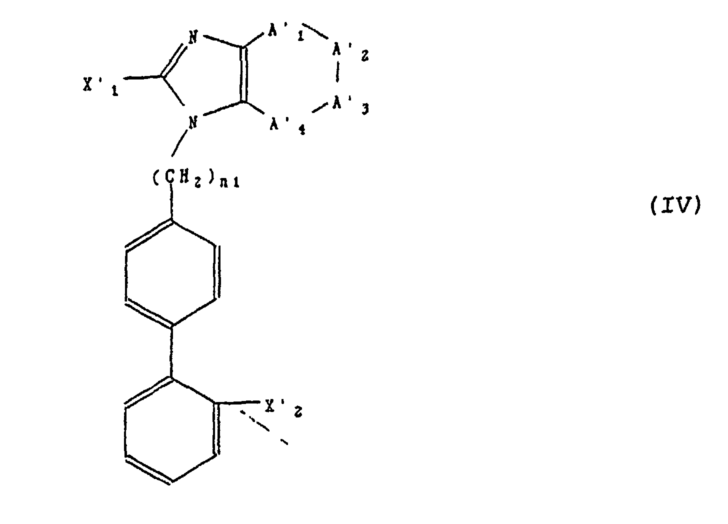 dans laquelle
X'₁, A'₁, A'₂, A'₃, A'₄, X'₂ et n₁ ont les significations indiquées ci-dessus,
produit de formule (IV) pouvant déjà constituer un produit de formule (I), que le cas échéant, l'on transforme pour obtenir un autre produit de formule (I) ou produit de formule (IV) que l'on transforme en produit de formule (I) en soumettant ce produit de formule (IV) ou ce produit de formule (I), si désiré et si nécessaire, à l'une ou plusieurs des réactions suivantes, dans un ordre quelconque :
   - élimination des groupements protecteurs que peuvent porter les fonctions réactives protégées,
   - salification par un acide minéral ou organique ou par une base minérale ou organique pour obtenir le sel correspondant,
   - estérification de fonction acide,
   - saponification de fonction ester en fonction acide,
   - transformation de fonction alkyloxy en fonction hydroxyle,
   - transformation de la fonction cyano en fonction acide,
   - réduction de la fonction carboxy en fonction alcool,
   - oxydation de l'atome de soufre en sulfoxyde ou en sulfone,
   - transformation de la fonction cétone en fonction oxime,
   - transformation de la fonction cétone en fonction alcool,
   - transformation de la fonction amine en fonction amide,
   - substitution par un atome d'halogène,
   - dédoublement des formes racémiques,
   lesdits produits de formule (I) ainsi obtenus étant sous toutes les formes isomères possibles racémiques, énantiomères et diastéréoisomères.

Dans des conditions préférentielles de mise en oeuvre de l'invention, le procédé ci-dessus est réalisé de la manière suivante :

Le produit de formule (IV) peut être obtenu par réaction du produit de formule (III) sur la fonction amine libre du produit de formule (II).

Cette réaction de condensation des produits de formule (II) avec un composé de formule (III) dans laquelle l'atome d'halogène représente de préférence un atome de brome peut être réalisée, par exemple, dans un solvant organique tel que le diméthylformamide ou le tétrahydrofuranne : ainsi le dérivé halogéné peut être condensé sur l'anion de l'imidazole de formule (II) préparé par exemple par action d'une base forte telle que l'hydrure de sodium ou de potassium ou encore d'un alcoolate de sodium ou de potassium tel que par exemple le méthylate de sodium ou le carbonate de potassium dans le diméthylformamide.

Comme indiqué ci-dessus, selon les valeurs de X'₁, A'₁, A'₂, A'₃, A'₄, R'₁, R'₂, X'₂ et n₁, les produits de formule (IV) constituent ou non des produits de formule (I), qui peuvent être le cas échéant, transformés en d'autres produits de formule (I).

Les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle, triméthylsilyle, dihydropyranne, méthoxyméthyle ou tétrahydropyrannyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals cycliques
ou non cycliques tels que le diméthyle ou diéthylcétal ou l'éthylène dioxycétal,
- les fonctions acides des produits décrits ci-dessus peuvent être, si désiré, amidifiées par une amine primaire ou secondaire par exemple dans du chlorure de méthylène en présence de chlorhydrate de 1-éthyl-3-(diméthylaminopropyl) carbodiimide à la température ambiante :

- les fonctions acides peuvent être protégées par exemple sous forme d'esters formés avec les esters facilement clivables tels que les esters benzyliques ou ter butyliques ou des esters connus dans la chimie des peptides.

L'élimination de ces groupements protecteurs est effectuée dans les conditions usuelles connues de l'homme du métier notamment l'hydrolyse acide effectuée avec un acide tel que l'acide chlorhydrique, benzène sulfonique ou paratoluène sulfonique, formique ou trifluoroacétique.

Le groupement phtalimido est éliminé par l'hydrazine. On trouvera une liste de différents groupements protecteurs utilisables par exemple dans le brevet BF 2 499 995.

Les produits décrits ci-dessus peuvent, si désiré, faire l'objet de réactions de salification par un acide minéral ou organique selon les méthodes usuelles connues de l'homme de métier.

Les produits décrits ci-dessus peuvent, si désiré, faire l'objet, sur les éventuelles fonctions carboxy, de réactions de salification par une base minérale ou organique ou d'estérification : ces réactions d'estérification et de salification peuvent être réalisées selon les méthodes usuelles connues de l'homme de métier.

Les éventuelles fonctions carboxy estérifiées des produits décrits ci-dessus peuvent être, si désiré, réduites en fonction alcool par les méthodes connues de l'homme de métier et notamment par de l'hydrure de lithium et d'aluminium dans un solvant tel que par exemple le tétrahydrofuranne ou encore le dioxanne ou l'éther éthylique.

Les éventuelles transformations de fonctions ester en fonction acide des produits décrits ci-dessus peuvent être, si désiré, réalisées dans les conditions usuelles connues de l'homme de métier notamment par hydrolyse acide ou alcaline par exemple par de la soude ou de la potasse en milieu alcoolique tel que, par exemple, dans du méthanol ou encore par de l'acide chlorhydrique ou sulfurique.

Les éventuelles fonctions cyano des produits décrits ci-dessus peuvent être, si désiré, transformées en fonction acide dans les conditions usuelles connues de l'homme de métier par exemple par une hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.

Les éventuelles fonctions alkoxy telles que notamment méthoxy des produits décrits ci-dessus peuvent être, si désiré, transformées en fonction alcool dans les conditions usuelles connues de l'homme de métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide acétique au reflux.

L'oxydation de l'éventuel atome de soufre en sulfoxyde ou en sulfone peut être effectuée au moyen d'un peracide par exemple, l'acide métachloroperbenzoïque.

La transformation de la fonction cétone en fonction oxime peut être effectuée par action d'un composé aminé par exemple le chlorhydrate d'hydroxylamine en présence de base faible celle que l'acétate de sodium.

La transformation de la fonction cétone en alcool peut être effectuée au moyen d'un organometallique par exemple un organozincique ou un organomagnésien.

La transformation de la fonction amine en amide peut être effectuée au moyen d'un isocyanate par exemple le propylisocyanate.

Les éventuelles réactions de substitution par un atome d'halogène et notamment les réactions de substitution de fonction hydroxyle ou mercapto par un atome d'halogène peuvent être, si désiré, réalisées dans les conditions usuelles connues de l'homme de métier telles que, par exemple par le chlorure de thionyle, le pentachlorure de phosphore (PCl₅), l'oxychlorure de phosphore POCl₃ dans un solvant tel que l'éther, le chlorure de méthylène ou le tétrahydrofuranne en présence ou non d'une base telle que la pyridine, ou par un tétrahalogène de méthane tel que le tétrachlorure ou le tétrabromure et la triphénylphosphine.

Les éventuelles formes optiquement actives des produits de formule (I) peuvent être préparées par dédoublement des racémiques selon les méthodes usuelles.

Les composés de formule (I) tels que définis ci-dessus ainsi que leurs sels d'addition avec les acides présentent d'intéressantes propriétés pharmacologiques.

Les produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes.

Ces propriétés justifient leur application en thérapeutique et l'invention a également pour objet à titre de médicaments, les produits tels que définis par la formule (I) ci-dessus, lesdits produits de formule (I) étant sous toutes les formes isomères possibles racémiques ou optiquement actives, ainsi que les sels d'addition avec les acides minéraux ou organiques pharmaceutiquement acceptables desdits produits de formule (I).

L'invention a tout particulièrement pour objet, à titre de médicaments, les produits décrits ci-après dans les exemples et notamment les produits de formule (I) suivants :
- le 4'-[(2-butyl-7-oxo-1,5,6,7-tétrahydro-thiopyrano[2,3-d] imidazol-1-yl) méthyl] N-[(diméthylamino) méthylène) (1,1'-biphényl) 2-sulfonamide,
- le 4'-[(2-butyl-7-oxo-1,5,6,7-tétrahydro-thiopyrano[2,3-d] imidazol-1-yl) méthyl] (1,1'-biphényl) 2-sulfonamide,
- le (2-(4'-((2-butyl-1,5,6,7-tétrahydro-7-oxo-thiopyrano[2,3-d] imidazol-1-yl) méthyl) (1,1'-biphényl) sulfonyl) carbamate d'éthyle,
- le N-(2-(4'-((2-butyl-1,5,6,7-tétrahydro-7-oxo-thiopyrano[2,3-d] imidazol-1-yl) méthyl) (1,1'-biphényl) sulfonyl) N'-propyl-urée,
- le 4'-[(2-butyl-7-(hydroxyimino)-1,5,6,7-tétrahydro-thiopyrano[2,3-d] imidazol-1-yl) méthyl] (1,1'-biphényl) 2-sulfonamide,
- le 4'-[(2-butyl-7-((((propylamino) carbonyl) oxy) imino)-1,5,6,7-tétrahydro-thiopyrano[2,3-d] imidazol-1-yl) méthyl] N- [(propylamino) carbonyl] (1,1'-biphényl) 2-sulfonamide,
- 4'-[(2-butyl 7-(hydroxyimino) 1,5,6,7-tétrahydro thiopyrano[2,3-d]imidazol-1-yl) méthyl] N-((propylamino) carbonyl) (1,1'-biphényl) 2-sulfonamide,
- le 4'-[(2-butyl-1,7-dihydro-7-oxo-thiopyrano[2,3-d]imidazol-1-yl) méthyl] N-[(diméthylamino) méthylène) (1,1'-biphényl) 2-sulfonamide,
- le 4'-[(2-butyl-1,7-dihydro-7-oxo-thiopyrano[2,3-d]imidazol-1-yl) méthyl] (1,1'-biphényl) 2-sulfonamide,
- le 4'-[(2-butyl-1,7-dihydro-7-oxo-thiopyrano[2,3-d]imidazol-1-yl) méthyl] N-[(propylamino) carbonyl) (1,1'-biphényl) 2-sulfonamide, et également le produit suivant acide 2-butyl 1-[[2'-[[(éthoxycarbonyl] amino] sulfonyl] (1,1'-biphényl)-4-yl] méthyl] 1,5,6,7-tétrahydrothiopyrano [2,3-d] imidazol 7-carboxylique, lesdits produits de formule (I) étant sous toutes les formes isomères possibles racémiques, énantiomères et diastéréoisomères, ainsi que les sels d'addition avec les acides minéraux et organiques ou avec les bases minérales et organiques, pharmaceutiquement acceptables, desdits produits de formule (I).

Les médicaments, objet de l'invention, peuvent être utilisés dans le traitement de l'hypertension artérielle, des insuffisances cardiaques, des insuffisances rénales et dans la prévention des resténoses post-angioplastie et notamment dans les accidents vasculaires cérébraux.

Ils peuvent également être utilisés dans le traitement de certains désordres gastro-intestinaux, gynécologiques et en particulier pour un effet relaxant au niveau de l'utérus.

L'invention s'étend aux compositions pharmaceutiques renfermant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Les composés de départ de formules (II) et (III) peuvent être préparés comme suit :

Le produit de départ de formule (II) peut être obtenu en faisant réagir un composé de formule (IIa) : 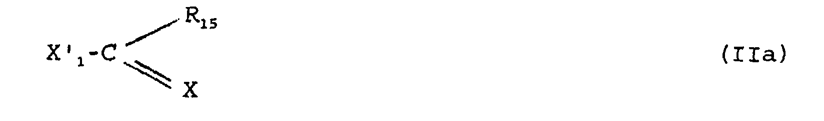
dans laquelle X représente un atome d'oxygène ou un radical NH, R₁₅ représente un radical hydroxyle, alkoxy, amino ou un atome d'halogène et X'₁ a la signification indiquée ci-dessus avec un composé de formule (IIb) : 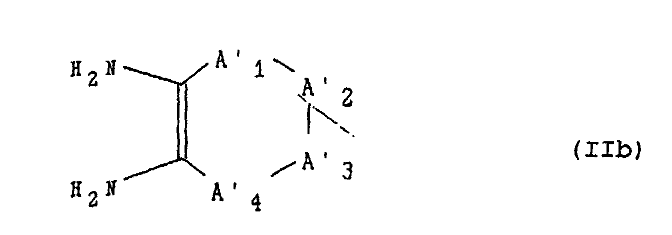
dans laquelle A'₁, A'₂, A'₃ et A'₄ ont les significations indiquées ci-dessus, pour obtenir le composé de formule (II).

La présente invention concerne aussi notamment un procédé de préparation du produit de formule (II) tel que défini ci-dessus, caractérisé en ce que l'on fait réagir un composé de formule (Fa) : 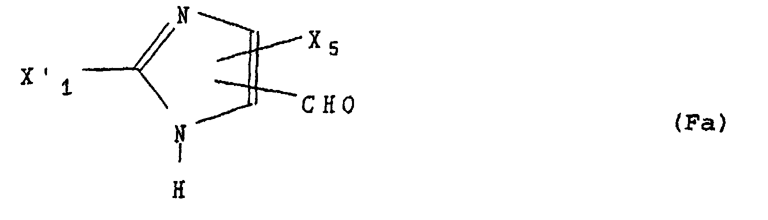 dans laquelle X₅ représente un radical alkyle, alcoxy, hydroxyle, formyle, alkylthio, phénylthio, benzylthio, carboxy libre, salifié ou estérifié ou amino, éventuellement substitués par un ou deux radicaux alkyle, tel que les radicaux alkyle et phényle de ces radicaux que peut comprendre ou représenter X₅ sont éventuellement substitués par un ou plusieurs radicaux choisis parmi les atomes d'halogène, les radicaux hydroxyle, alkyle, aralcoxy, et sont, si nécessaire, protégés,
avec un halogénure de lithium ou de magnésium de formule (Fb) :

A"₂----A"₃-M Hal (Fb)

dans laquelle Hal représente un atome d'halogène. M représente un atome de lithium ou de magnésium et A"₂----A"₃ représente soit un radical éthényle non substitué ou substitué par 1, 2 ou 3 radicaux R'₁ et R'₂ tels que définis ci-dessus, dans lesquels les éventuelles fonctions réactives sont éventuellement protégées soit l'un de A"₂ et A"₃ représente un radical amino éventuellement substitué par un ou 2 radicaux R'₁ et R'₂ tels que définis ci-dessus, dans lesquels les éventuelles fonctions réactives sont éventuellement protégées ci-dessus, pour obtenir un produit de formule (Fc) : 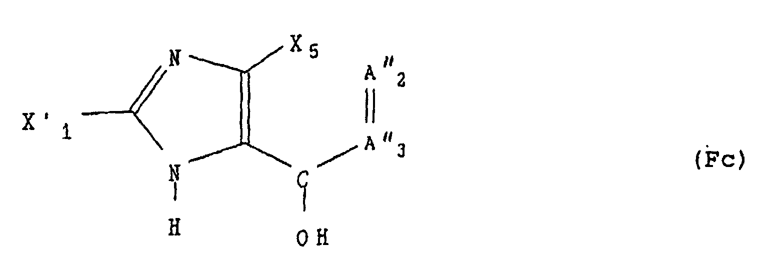 dans laquelle X'₁, X₅, A"₂ et A"₃ ont les significations indiquées ci-dessus, que l'on oxyde pour obtenir un composé de formule (Fd) : 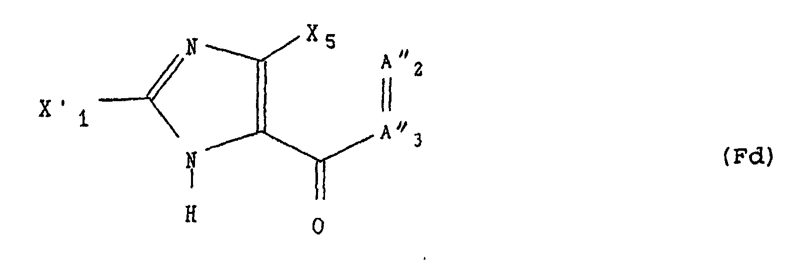 dans laquelle X'₁, X₅, A"₂ et A"₃ ont les significations indiquées ci-dessus, que l'on soumet à une réaction de cyclisation pour obtenir le produit de formule (Fe) : 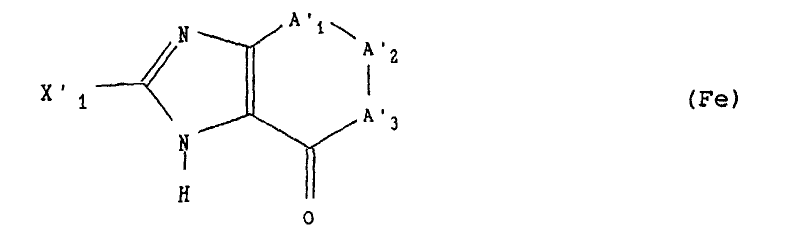 dans laquelle X'₁, A'₁, A'₂ et A'₃ ont les significations indiquées ci-dessus, produits de formule (Fe) pouvant constituer un produit de formule (II) que le cas échéant l'on transforme pour obtenir un autre produit de formule (II) ou produit de formule (Fe) que l'on transforme en produit de formule (II) en le soumettant, si désiré et si nécessaire, à l'une ou plusieurs des réactions suivantes, dans un ordre quelconque :
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction d'estérification de fonction acide,
- une réaction de saponification de fonction ester en fonction acide,
- une réaction de transformation de fonction alkyloxy en fonction hydroxyle,
- une réaction de transformation de la fonction cyano en fonction acide,
- une réaction de transformation de la fonction cétone en fonction cyano,
- une réaction de réduction de la fonction carboxy en fonction alcool,
- une réaction de dédoublement des formes racémiques,
lesdits produits de formule (II) ainsi obtenus étant sous toutes les formes isomères possibles racémiques, énantiomères et diastéréoisomères.

On peut noter que :
- lorsque X₅ représente un radical formyl, alkylthio, phénylthio ou benzylthio, ces radicaux étant, si nécessaire protégés, on peut ainsi préparer les produits de départ de formule (II) telle que définie ci-dessus, dans laquelle A'₁ et A'₂ différents l'un de l'autre, sont choisis parmi l'atome de soufre, le radical -CH₂- ou un radical aminé, ces radicaux étant éventuellement substitués par un radical alkyle, phényle ou benzyle, ces radicaux étant eux-mêmes substitués ainsi qu'il est indiqué ci-dessus,
- lorsque X₅ représente un radical hydroxyle ou alcoxy, ces radicaux étant, si nécessaire protégés, on peut ainsi préparer les produits de départ de formule (II) telle que définie ci-dessus, dans laquelle A'₁ représente un atome d'oxygène.

De telles préparations sont illustrées dans les exemples ci-après.

Le composé de formule (II) tel que défini ci-dessus, peut notamment représenter le composé de formule (V) : 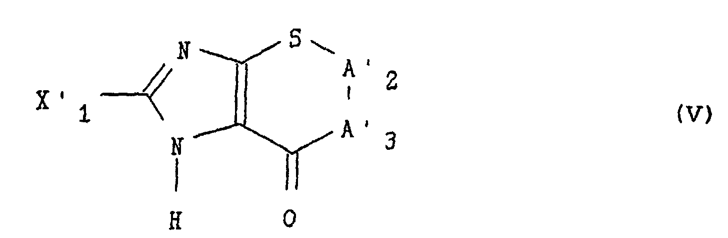 ou encore le composé de formule (VI) :

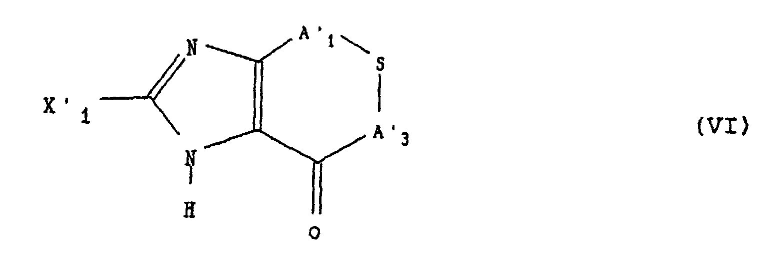

Les schémas ci-après 1, 2, 3 et 4 donnent des exemples de préparation de tels composés de formules (V) et (VI)

Dans les schémas suivants 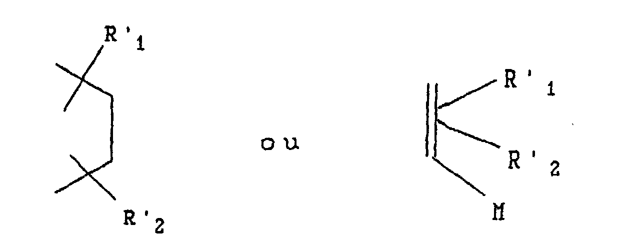 signifie que le radical hydrocarboné peut être le cas échéant non substitué ou substitué sur chacun des 2 carbones par 1 ou 2 radicaux R'₁ et R'₂

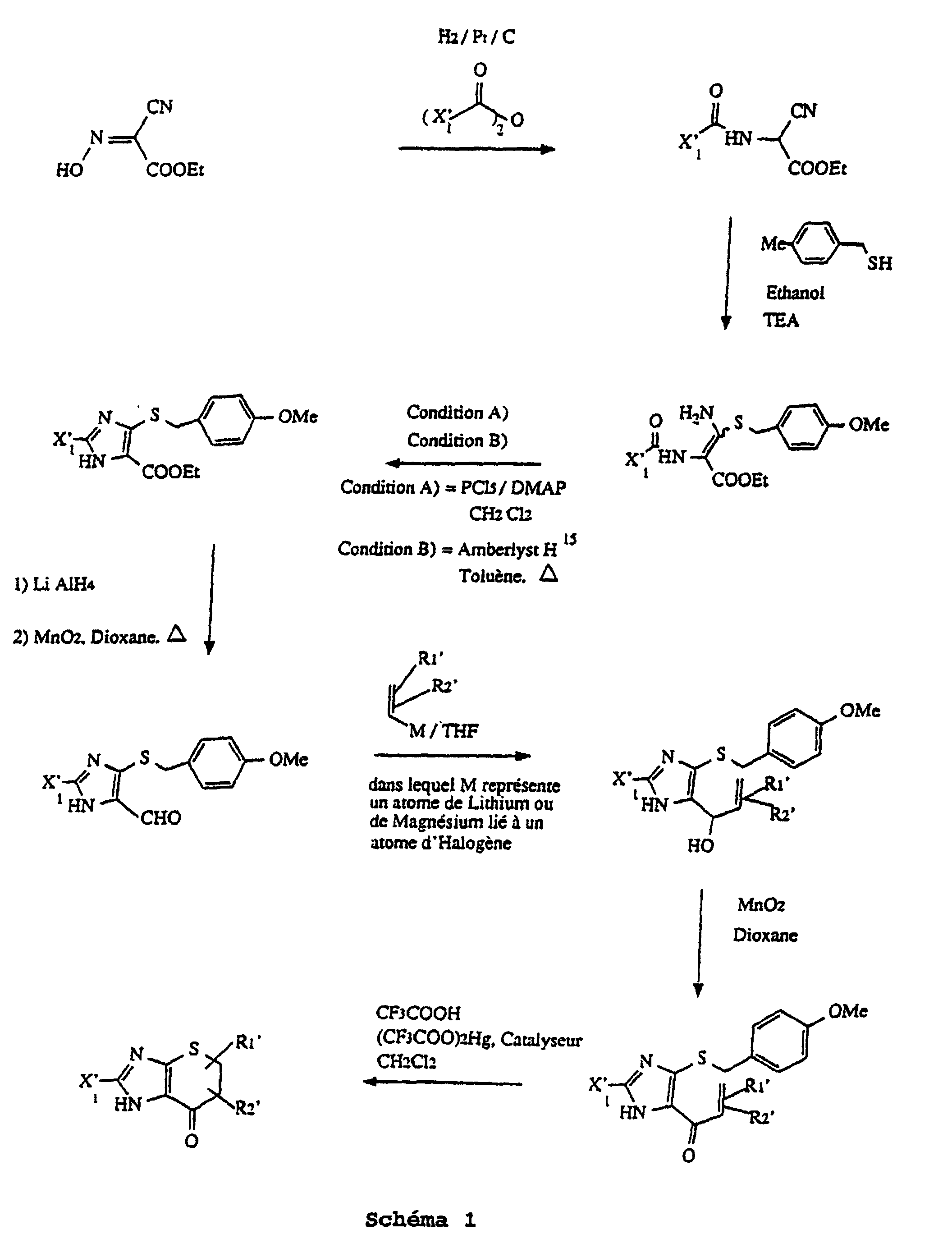

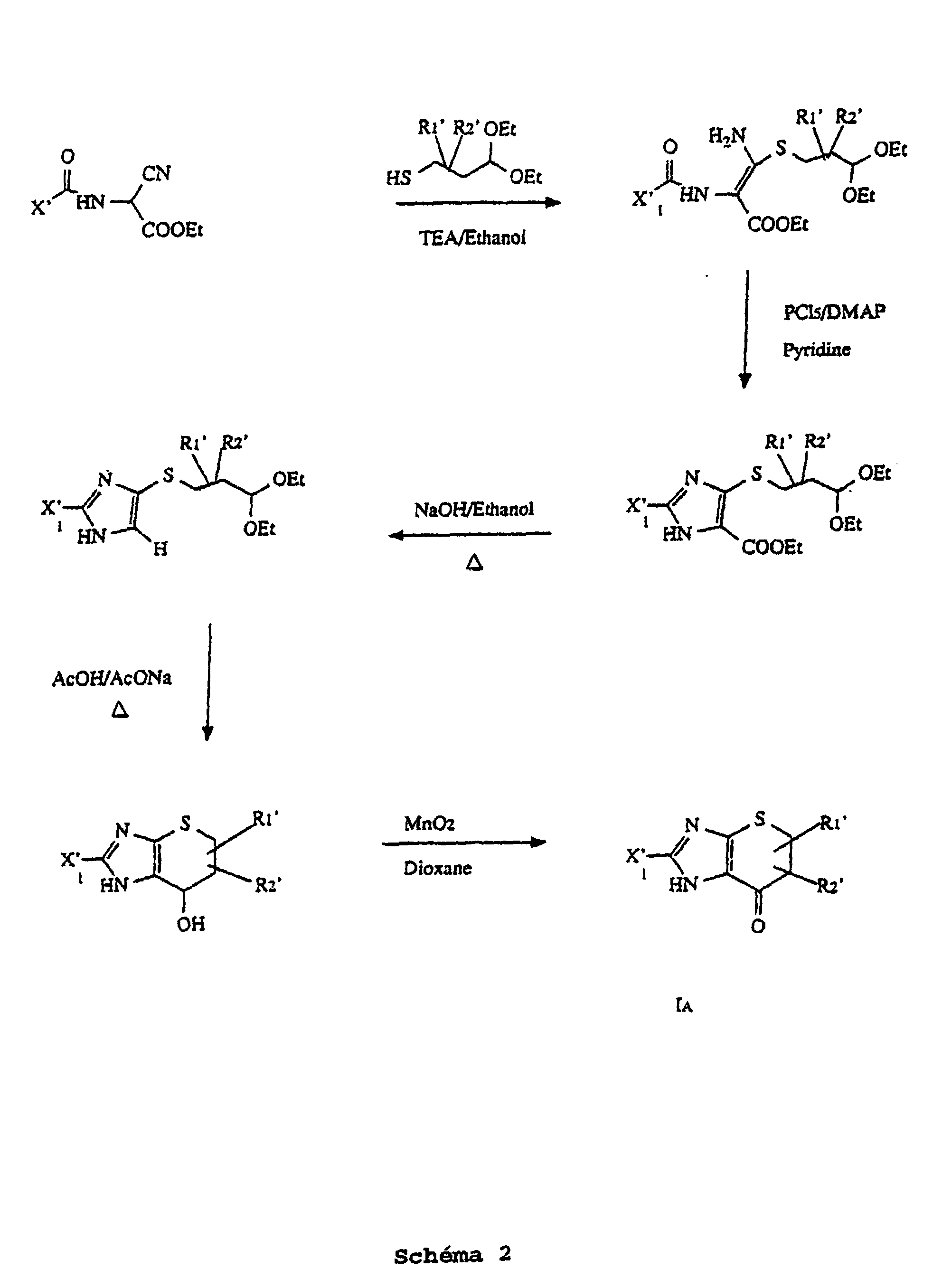

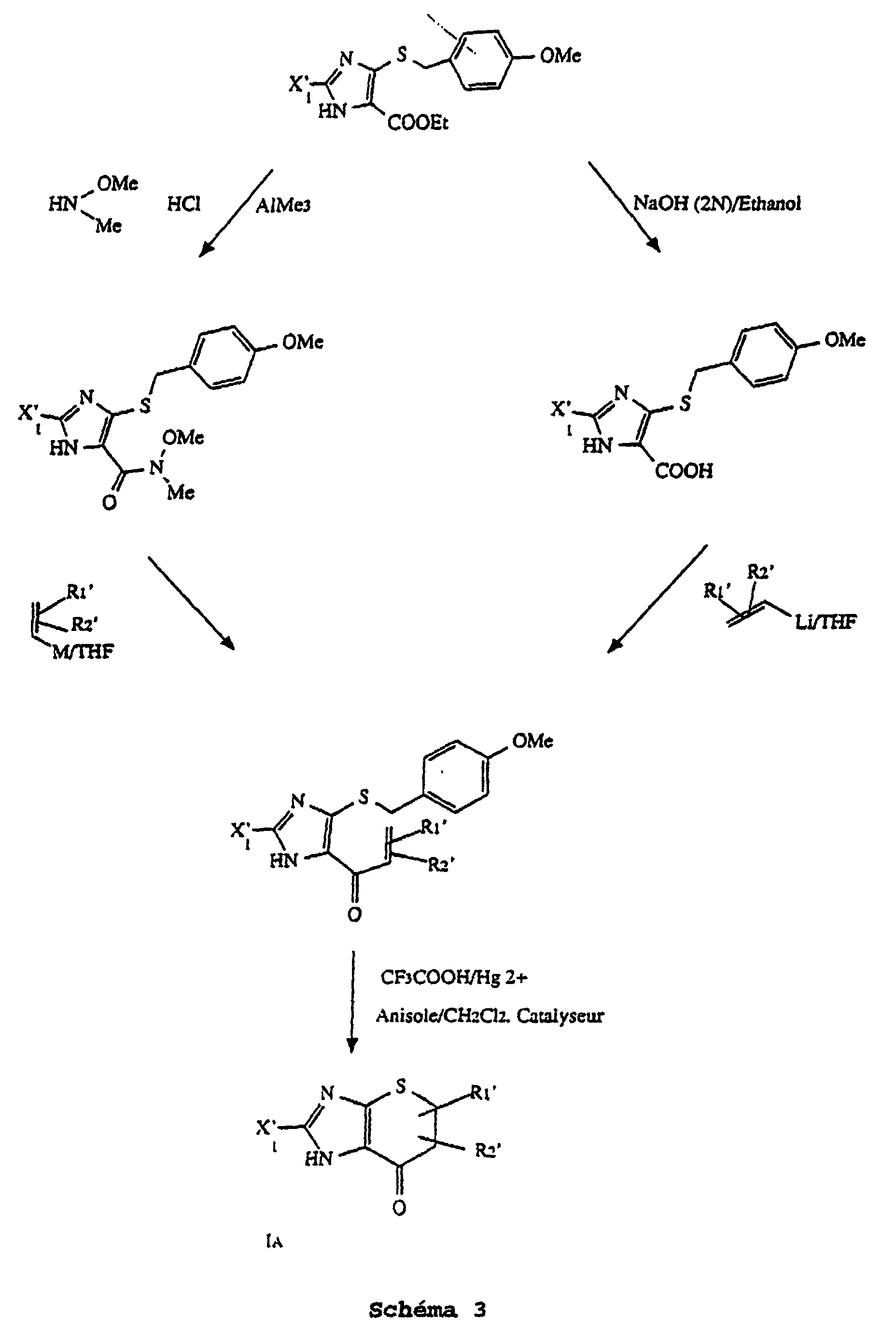

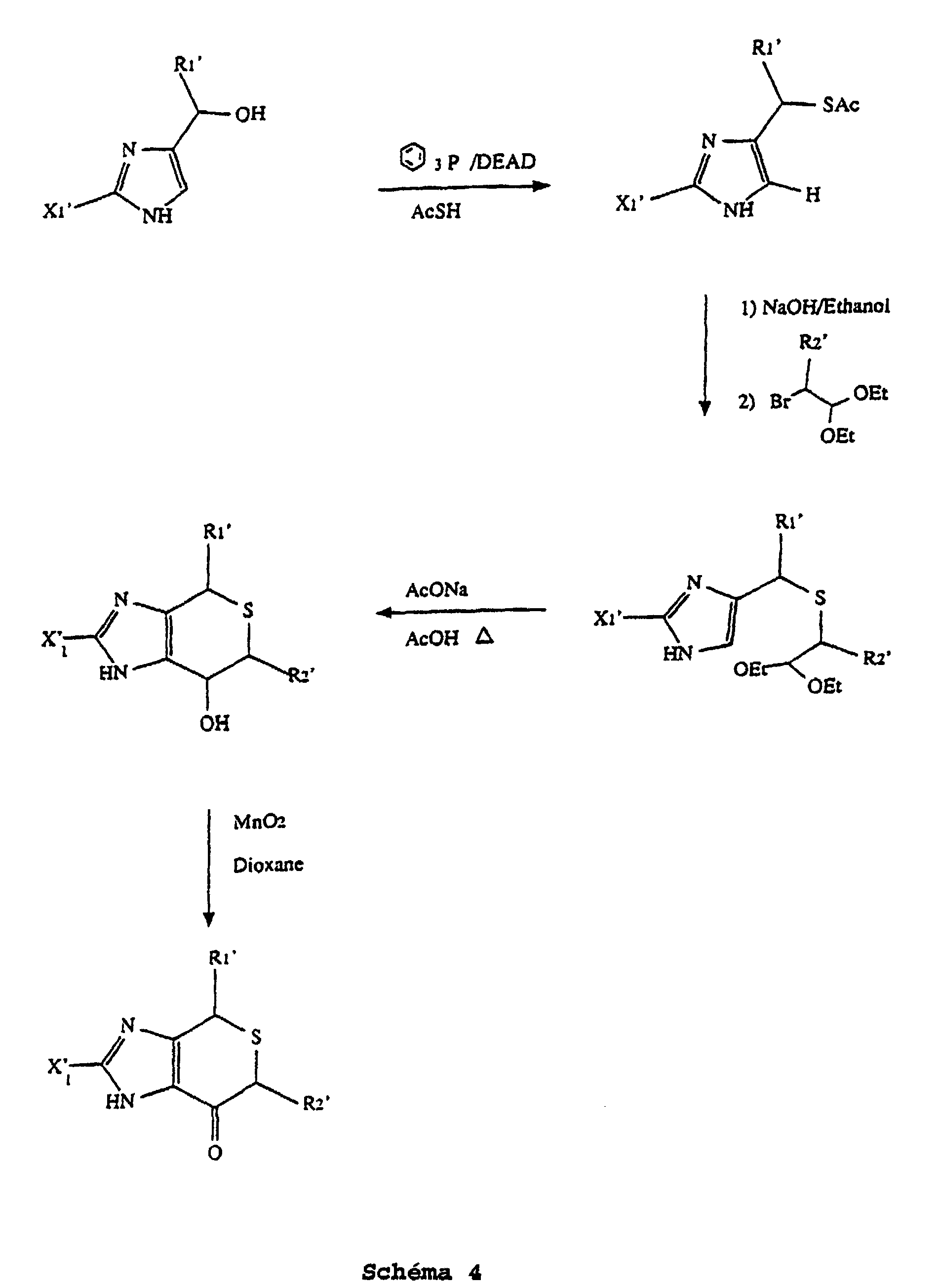

Un procédé de préparation de certains produits de formule (III) telle que définie ci-dessus peut consister à soumettre le composé de formule (IIIa) : 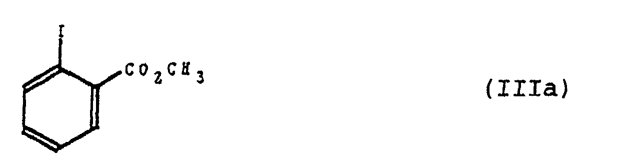 soit le iodobenzoate de méthyle que l'on peut trouver, par exemple, sous forme de produit commercialisé par JANSSEN, à l'action d'un composé de formule (IIIb) : 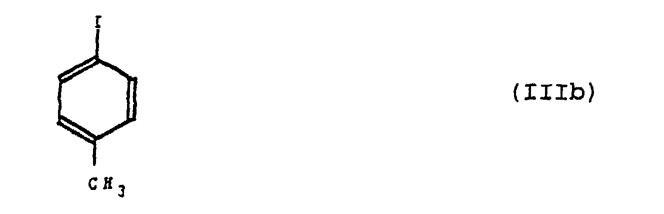 soit du iodotoluène que l'on peut trouver, par exemple, sous forme de produit commercialisé par FLUKA, la réaction se réalisant par exemple en présence de cuivre en poudre à une température d'environ 100°C à 300°C, pour obtenir le produit de formule (IIIc) : 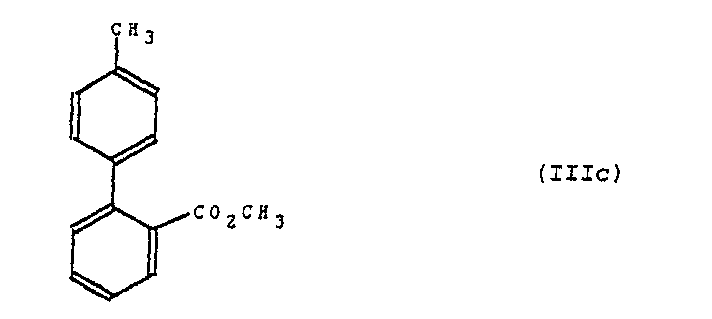 dont le radical carboxy estérifié peut, si désiré, être libéré du radical alkyle par les méthodes classiques connues de l'homme du métier ou indiquées ci-dessus, par exemple d'hydrolyse acide ou alcaline, le radical carboxy estérifié ou le radical carboxy libre obtenu après libération du radical alkyle pouvant être soumis à des réactions de réduction, addition ou substitution dans un ordre quelconque, ces réactions pouvant être réalisées par exemple selon les méthodes classiques connues de l'homme de métier, produit de formule (IIIc) que l'on peut soumettre à une réaction de bromation sur le radical méthyle par les méthodes classiques connues de l'homme de méthier par exemple par action du n-bromosuccinimide dans le tétrachlorure de carbone, puis à une réaction d'addition de la fonction amine dans les conditions indiquées dans la référence U. Ragnarsson, L. Grehn, Acc. Chem. Res. 1991, 24, 285-89 et ref. citées, afin d'obtenir à partir du produit de formule (IIIc) indiqué ci-dessus des composés de formule (III) tels que définis ci-dessus.

En opérant de la même façon que décrit précédemment, au départ de produits de formules : 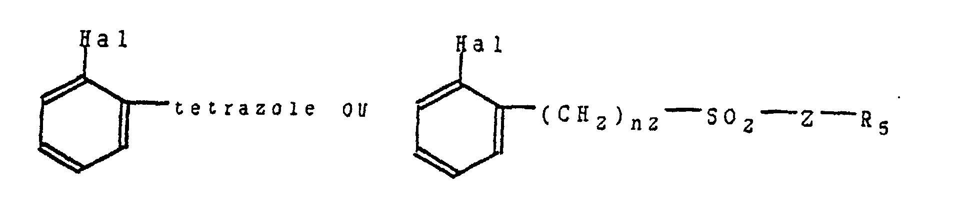 dans lesquels Hal représente un atome d'halogène qui peut être par exemple un atome de brome ou de préférence un atome d'iode, on obtient ainsi d'autres composés de formule (III).

Des exemples de préparation de composés de formule (III) sont décrits dans la littérature et des exemples en sont donnés notamment dans le brevet US 4,880,804 et dans les demandes de brevet européens EP 0.400.974 et 0.461.040.

Les composés de départ de formules (IIa), (IIb), (IIIa) et (IIIb) peuvent être disponibles dans le commerce ou préparés selon les méthodes usuelles connues de l'homme du métier.

Les produits de formule (Fa) utilisés au départ du procédé sont des produits connus ou peuvent être préparés à partir de l'alcool ou de l'ester correspondant selon des conditions opératoires connues de l'homme du métier.

La présente invention a enfin pour objet à titre de produits industriels nouveaux, et notamment à titre de produits intermédiaires nécessaires à la préparation des produits de formule (I), les composés de formules (II) et (III).

En plus des produits décrits dans les exemples qui illustrent l'invention sans toutefois la limiter, les produits suivants constituent des produits pouvant être obtenus dans le cadre de la présente invention .
Dans les produits ci-dessous, Ar représente le radical : 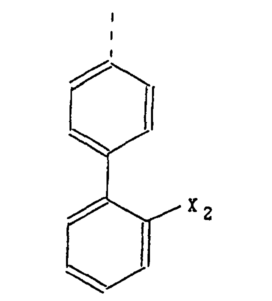 dans lequel X₂ a la signification indiquée ci-dessus et peut représenter notamment un radical cyano, carboxy libre salifié
ou estérifié, tétrazolyle éventuellement substitué ou salifié et (CH₂)ₙ₂-SO₂-Z-R₅ tel que défini ci-dessus représentant notamment :

-SO₂-NH-CO-NH-CH₂-CH₂-CH₃

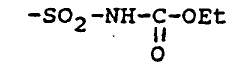

-SO₂-NH-CO-NH-CH₂-CH=CH₂

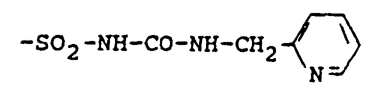

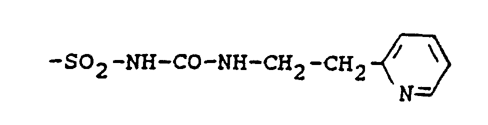

-NH-SO₂ -CF₃

Dans les produits ci-après, S peut être remplacé par : O ; NH ; NR₁ avec R₁ tel que défini ci-dessus et n représente 0, 1 ou 2.

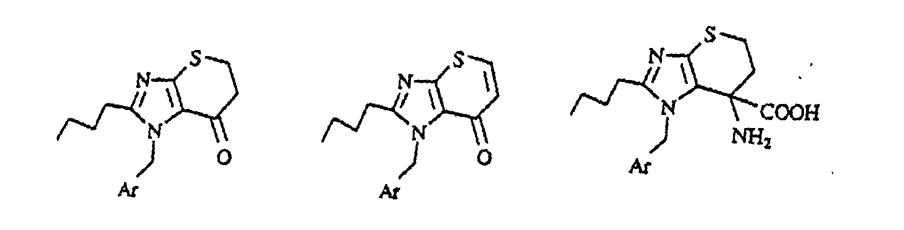

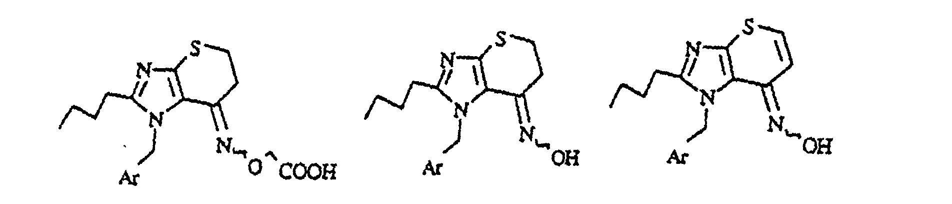

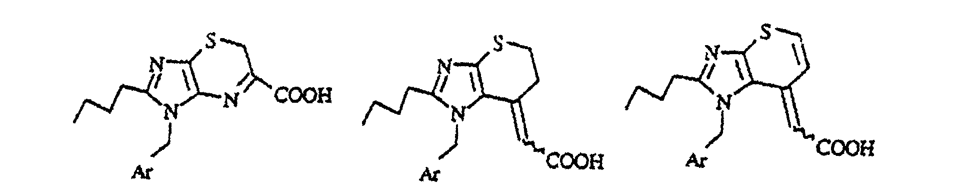

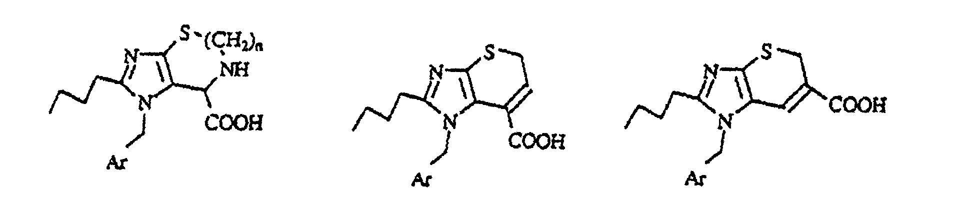

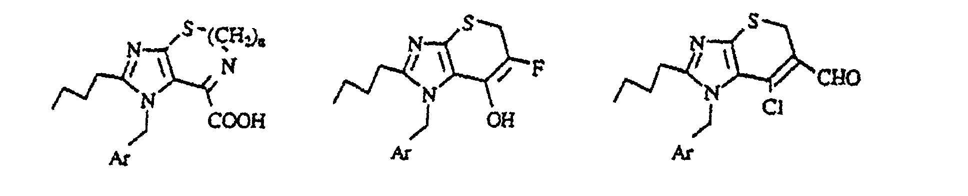

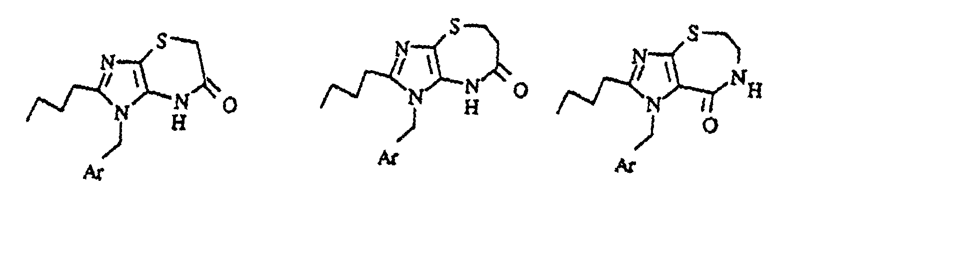

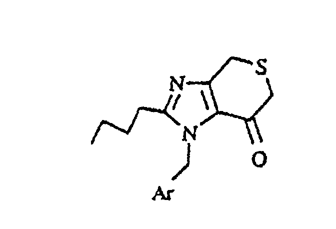

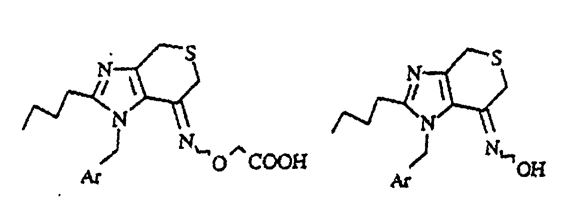

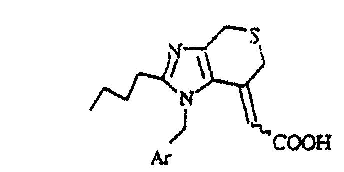

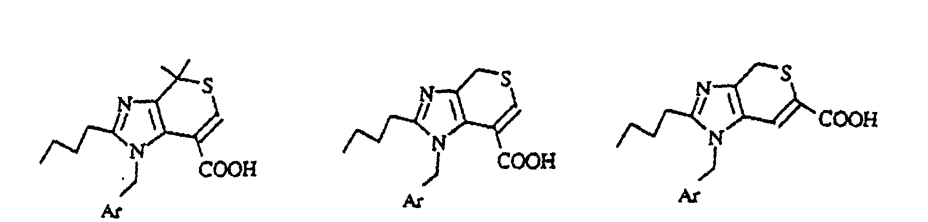

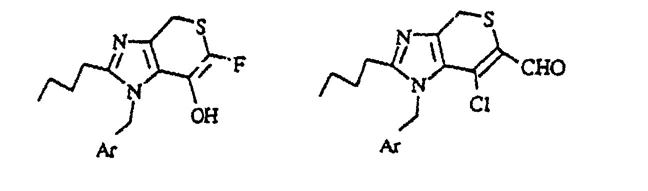

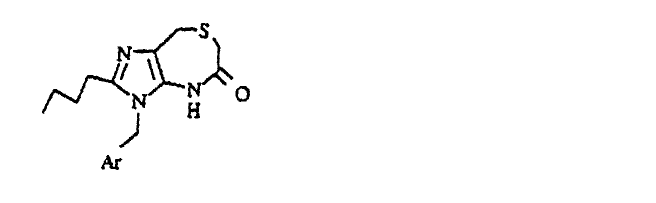

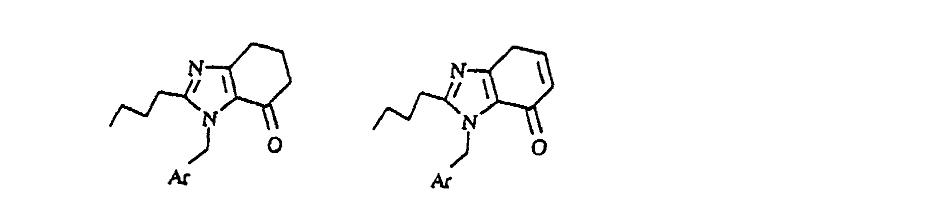

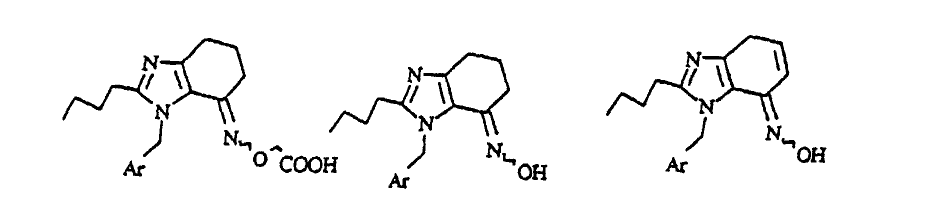

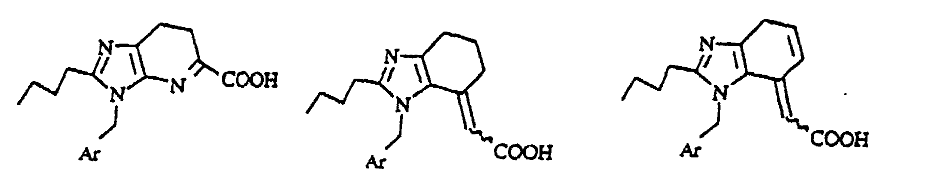

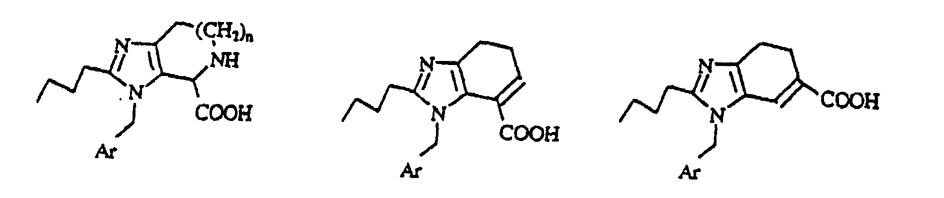

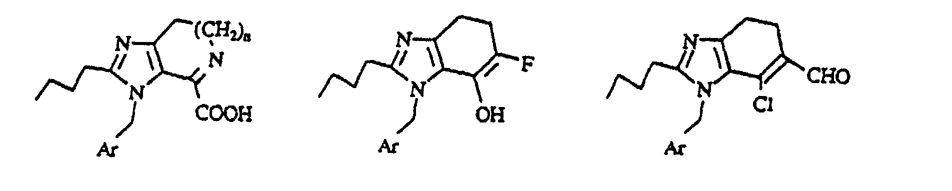

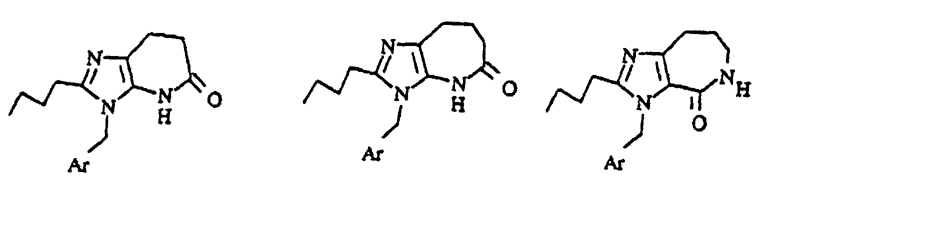

On peut citer encore d'autres molécules préférées : 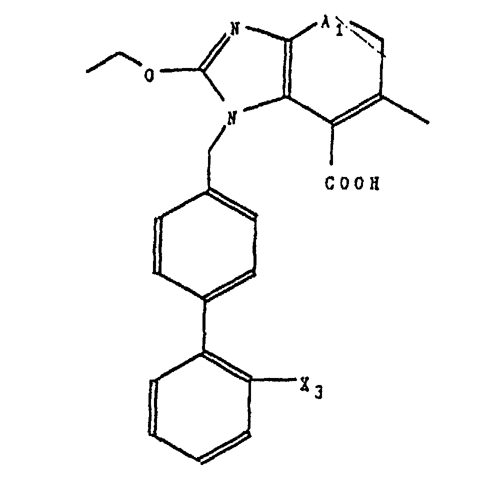 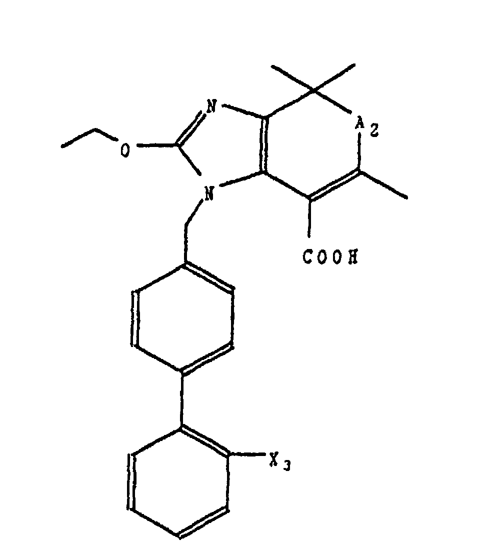 dans lesquelles A₁ et A₂ représentent un atome de soufre ou un atome d'oxygène et X₃ représente : 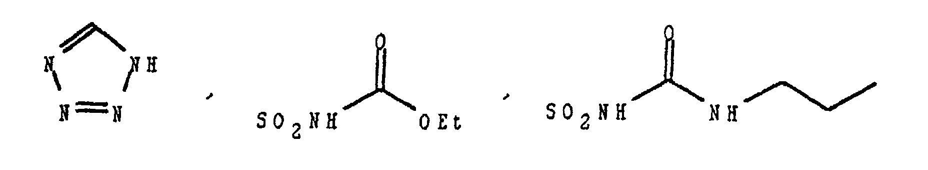 ou NH-SO₂-CF₃.

Les exemples suivants illustrent l'invention sans toutefois la limiter.

### EXEMPLE 1 : 4'-[(2-butyl-6,7-dihhydro 7-oxo-thiopyrano (2,3-d)imidazol-1(5H)-yl)) méthyl] N-[(diméthylamino) méthylène) (1,1'-biphényl) 2-sulfonamide

### Stade 1 : 2-butyl 5-[((4-méthoxyphényl) méthyl) thio] 1H-imidazole 4-méthanol

On solubilise 50 g soit 143,5 mmol de 2-butyl 4-[((4-méthoxyphényl) méthyl) thio] 1H-imidazole 5-carboxylate d'éthyle préparé comme indiqué dans la demande de brevet européen n° 0 465 368, dans 300 ml de chlorure de méthylène. On porte le milieu sous agitation à -78°C et additionne le DIBAH 1,2N dans le toluène (400 ml-68,3 g-480 mmol), puis on laisse le milieu revenir à température ambiante pendant environ 30 minutes. On hydrolyse le milieu réactionnel par 130 ml d'eau, filtre, lave par 500 ml de chlorure de méthylène puis par 3 fois 500 ml d'une solution chlorure de méthylène/méthanol (9-1).

On porte à sec, on récupère 100 g (solide orange) et ajoute 500 ml de chlorure de méthylène/méthanol (9-1), filtre l'insoluble blanc et porte à sec la phase organique.

On obtient 46,55 g (poudre jaune orangée) de produit que l'on purifie sur colonne de silice avec pour éluant méthanol/chlorure de méthylène (5-95).

On récupère 32,49 g de produit attendu.

### ANALYSES :

### IR, cm⁻¹, nujol

absence de C=O
absorption région NH/OH
hétérocycle + aromatique : 1610, 1580, 1510 cm⁻¹

### Stade 2 : 2-butyl 5-[((4-méthoxyphényl) méthyl) thio] 1H-imidazole 4-carboxaldéhyde

On solubilise 32,51 g soit 106 mmol du produit obtenu au stade 1, dans 250 ml de dioxanne. Puis sous agitation, on additionne MnO₂ en poudre (46,1 g-530,5 mmol). Le milieu réactionnel ainsi formé est chauffé à 50°C, et agité. Après environ 7 heures de chauffage à 50°C, on ajoute 18,44 g de MnO₂ et on maintient à 50°C. On laisse refroidir le milieu réactionnel une nuit à température ambiante. On ajoute 750 ml d'acétate d'éthyle, agite, puis filtre et porte à sec. On récupère 33,45 g (poudre rose) que l'on purifie sur silice (éluant acétate d'éthyle/flugène (1-1)). On récupère 28,77 g de produit attendu.

### ANALYSES :

| IR, cm⁻¹, CHCl₃ | |
|---|---|
| =NH | 3424, 3221 cm⁻¹ |
| C=O | complexe ∼ 1640 cm⁻¹ |
| hétérocycle + aromatique | 1611, 1585, 1540, 1513, 1497 cm⁻¹ |

### Stade 3 : 2-butyl α-éthényl 5-[((4-méthoxyphényl) méthyl) thio] 1H-imidazole 4-méthanol

On solubilise 28,77 g soit 94,5 mmol du produit obtenu au stade 2, dans 180 ml de THF, porte le mélange réactionnel ainsi formé à -78°C et introduit sous agitation 37,22 g soit 283,5 mmol de bromure de vinylmagnésium, laisse la réaction sous agitation environ 1 heure à une température d'environ - 10°C. On hydrolyse le milieu réactionnel à environ -10°C puis ajoute 150 ml de méthanol. Puis on ajoute 500 ml d'une solution aqueuse glacée saturée en chlorure d'ammonium. Ensuite on extrait par 3 fois 250 ml d'un mélange composé de méthanol/chlorure de méthylène (1-9). Les phases organiques sont réunies, lavées par 300 mml d'eau puis séchées. Après filtration et évaporation, on obtient 41,94 g (poudre jaune orangée). On solubilise la poudre dans l'acétate d'éthyle à chaud puis à température ambiante, filtre, rince à l'acétate d'éthyle puis à l'éther et sèche. On obtient 24,11 g (poudre blanche) de produit attendu.

### ANALYSES :

### IR, cm⁻¹, CHCl₃

Absence de C=O
OH complexe 3600-3550 cm⁻¹
=C-NH 3440 cm⁻¹
C=C aromatique et hétéroaromatique : 1640, 1610, 1583, 1510 cm⁻¹ avec -CH=CH₂ possible à 930 cm⁻¹.

### Stade 4 : 1-[2-butyl 4 -[((4-méthoxyphényl) méthyl) thio] 5-imidazolyl] 2-propèn-1-one

On dissout 3 g du produit obtenu au stade 3 à 30°C dans 60 ml de dioxanne. On laisse revenir à température ambiante et ajoute 1,56 g (2 équivalents) de MnO₂ environ toutes les 30 minutes pendant environ 2 heures, soit 10 équivalents en tout. On verse dans 500 ml d'AcOEt, filtre et porte à sec le. filtrat. On récupère 2,7 g de solide jaune. On chromatographie sur silice avec AcOEt/flugène (1-2). On récupère 2,1 g de produit attendu (solide jaune vif).

### ANALYSES :

### IR, CHCl₃

NH 3425, 3220 cm⁻¹
carbonyle 1635 cm⁻¹
hétérocycle, aromatique C=C 1610, 1585, 1533, 1513, 1487 cm⁻¹

### Stade 5 : 2-butyl 5,6-dihydro thiopyrano[2,3-d]imidazol 7(1H)-one

On dissout 2,1 g du produit obtenu au stade 4 (6,4 mmoles) dans 65 ml de dichlorométhane (10 ml/mmole). On introduit à environ 0°C :
- 25 ml d'anisole (4 ml/mmole)
- 12,5 ml d'acide trifluoroacétique (2 ml/mmole)
- 271 mg de trifluoroacétate mercurique (10 % molaire).

On laisse revenir à environ 20°C et on agite environ 5 heures. On porte à sec à environ 45°C et ajoute 50 ml de solution saturée de NaHCO₃ (jusqu'à pH = 8-9). On ajoute 100 ml d'eau et on extrait avec CH₂Cl₂ (3 fois). On sèche et obtient 3,19 g de solide jaune. On empâte dans du cyclohexane et obtient 1,376 g du produit attendu (poudre jaune clair).

### ANALYSES :

### IR, CHCl₃

=C-NH 3430, 3196 cm⁻¹
carbonyle 1637 cm⁻¹
C=C, C=N 1533, 1497 cm⁻¹.

### Stade 6 : 4'-[(2-butyl-6,7-dihhydro 7-oxo-thiopyrano (2,3-d)imidazol-1(5H)-yl)) méthyl] N-[(diméthylamino) méthylène) (1,1'-biphényl) 2-sulfonamide

On introduit dans 882,8 mg (1 équivalent) de produit obtenu au stade 5, dissous dans 60 ml de diméthylformamide, 764 mg (1,3 équivalent) de carbonate de potassium. On laisse agiter environ 5 minutes et ajoute 2,08 g de 4'-(bromométhyl) N-[(diméthylamino) méthylène] (1,1'-biphényl) 2-sulfonamide dissous dans 70 ml de DMF. On laisse agiter environ 21 heures. On porte à sec et on extrait (eau/AcOEt) 3 fois. On porte à sec la phase organique et obtient 2,77 g de poudre jaune. On chromatographie sur silice dans l'AcOEt et récupère 1,91 g du produit attendu (solide blanc/jaune).

### ANALYSES :

### IR, CHCl₃

1628 cm⁻¹, absorption forte : cétone + -SO₂-N=C-N
SO₂ : 1347, 1150 cm⁻¹

### EXEMPLE 2 : 4'-[(2-butyl-6,7-dihydro 7-oxo-thiopyrano (2,3-d) imidazol-1-(5H)-yl) méthyl] (1,1'-biphényl) 2-sulfonamide

On introduit 1,5 g du produit de l'exemple 1, 15 ml (10 volumes) d'éthanol, 4,5 ml (3 volumes) de HCl concentré. On laisse agiter environ 3 à 4 heures au reflux.

On basifie par de la soude 2N (jusqu'à pH=8-9), on ajoute 200 cc d'eau et extrait 3 fois par le dichlorométhane. La phase organique est lavée à l'eau, et portée à sec. On obtient 1,06 g de solide jaune. On purifie sur silice, éluant AcOEt/cyclohexane (5-5) et récupère 760 mg de produit attendu (solide blanc/jaune).

### ANALYSES :

### IR, CHCl₃

SO₂NH₂ 3450, 3350 cm⁻¹
carbonyle 1644 cm⁻¹
aromatique, hétéroaromatique, NH₂ déf. 1543, 1520, 1496 cm⁻¹.

### EXEMPLE 3 : 4'-[(2-butyl-6,7-dihydro 7-oxo-thiopyrano (2,3-d) imidazol-1-(5H)-yl) méthyl] N-[(propylamino) carbonyl] (1,1'-biphényl) 2-sulfonamide

On introduit 360 mg (1 équivalent) de produit de l'exemple 2, dissous dans 10 ml de DME. On ajoute 224 mg (2 équivalents) de K₂CO₃ et agite environ une heure à température ambiante avant d'ajouter 0,110 ml (1,5 équivalent) de propylisocyanate. On chauffe à 85°C pendant 3 à 4 heures. On ajoute 5 ml d'eau, acidifie (jusqu'à pH=3) avec HCl 1N (2 ml), extrait 3 fois avec CH₂Cl₂. On lave la phase organique avec une solution saturée de NaCl et porte à sec. On obtient 466,2 mg de solide blanc brut. On purifie sur silice, éluant CH₂Cl₂/AcOEt (2-1). On récupère 316 mg de produit attendu (solide blanc).

### ANALYSES :

### IR, CHCl₃

=C-NH 3371, 3404 cm⁻¹
carbonyle 1716, 1640 cm⁻¹
aromatique, hétéroaromatique, amide 1593, 1540, 1493 cm⁻¹.

| Microanalyse : | | | | |
|---|---|---|---|---|
| | C | H | N | S |
| % calculés | 59,977 | 5,965 | 10,361 | 11,86 |
| % trouvés | 59,8 | 6,1 | 10,1 | 11,8 |

### EXEMPLE 4 : 2-(4'-((2-butyl 6,7-dihydro 7-oxo-thiopyrano[2,3-d]imidazol-1-(5H)-yl) méthyl) (1,1'-biphényl) sulfonyl) carbamate d'éthyle

On introduit 378 mg (1 équivalent) du produit de l'exemple 2, dissout dans 10 cc de DME et 235,7 mg (2 équivalents) de K₂CO₃. On laisse agiter environ 1 heure, puis ajoute 0,32 ml (4 équivalents) de chloroformiate d'éthyle. On laisse agiter environ 21 heures à température ambiante, puis environ 1 heure à 60°C. On ajoute 10 cc de solution saturée de NaHCO₃ (jusqu'à pH = 6-7), et on extrait 3 fois avec l'AcOEt.

On lave la phase organique avec NaCl en solution saturée et porte à sec. On obtient 485 mg de solide jaune clair. On purifie sur silice (éluant AcOEt-cyclohexane (7-3)) et obtient 295 mg de produit attendu (solide jaune).

### ANALYSES :

### IR, CHCl₃

=C-NH 3385 cm⁻¹
carbonyle 1750, 1641 cm⁻¹
aromatique, hétéroaromatique, amide 1594, 1564, 1514, 1493 cm⁻¹.

| Microanalyse : | | | | |
|---|---|---|---|---|
| | C | H | N | S |
| % calculés | 59,183 | 5,539 | 7,963 | 12,153 |
| % trouvés | 59,0 | 5,8 | 7,8 | 11,8 |

### EXEMPLE 5 : 4'-[(2-butyl-7-(hydroxyimino)-1,5,6,7-tétrahydro-thiopyrano[2,3-d] imidazol-1-yl) méthyl] (1,1'-biphényl) 2- sulfonamide

On introduit 1 g de produit de l'exemple 2 (soit 2,19 mmoles), 20 cm³ d'éthanol, 5 cm³ de méthanol. On introduit d'un coup, 0,538 g d'acétate de sodium (6,57 mmoles), 0,460 g de chlorhydrate d'hydroxylamine (6,67 mmoles). On porte au reflux, pendant environ 48 heures. On rajoute 0,304 g de chlorhydrate d'hydroxylamine (4,37 mmoles, 2 eq). On laisse sous agitation pendant environ 48 heures. On filtre le précipité formé, lave à l'eau et sèche. On obtient 0,93 g de produit. Point de fusion > 200°C.

### ANALYSES :

### Spectre IR dans Nujol

NH₂ + OH 3390, 3280 cm⁻¹
SO₂ 1330, 1160 cm⁻¹
aromatique 1655 cm⁻¹
hétérocycles 1628 cm⁻¹
C=N 1590, 1560, 1548, 1535, 1502 cm⁻¹

### EXEMPLE 6 : 4'-[(2-butyl-7-((((propylamino) carbonyl) oxy) imino)-1,5,6,7-tétrahydro-thiopyrano[2,3-d] imidazol-1-yl) méthyl] N-[(propylamino) carbonyl] (1,1'-biphényl) 2-sulfonamide

On introduit 0,6 g de produit de l'exemple 5 (1,27 mmoles), 9 cm³ de diméthoxyéthane (15 vol), 0,444 g de carbonate de potassium (3,17 mmoles), 0,298 cm³ de propylisocyanate (2,5 eq 3,17 mmoles).

On laisse environ 1 heure sous agitation. On rajoute 0,06 cm³ de propylisocyanate. On laisse environ une demi-heure. On verse dans 20 cm³ d'eau plus 10 cm³ de solution saturée de chlorure d'ammonium. On extrait avec 3 fois 20 cm³ de chlorure de méthylène. On sèche, filtre et sèche. On obtient 0,88 g de produit que l'on purifie, par chromatographie sur silice. Le produit brut est déposé en solution dans un volume de l'éluant acétate d'éthyle, sur silice. On élue, sèche et obtient 0,325 g de produit attendu (poudre blanche).

### EXEMPLE 7 : 4'-[(2-butyl 7-(hydroxyimino) 1,5,6,7-tétrahydro thiopyrano[2,3-d]imidazol-1-yl) méthyl] N-((propylamino) carbonyl) (1,1'-biphényl) 2-sulfonamide

On introduit 283 mg de produit de l'exemple 6 (0,44 mmole), 3 cm³ d'éthanol (10 vol), 0,44 cm³ de soude 2N (2 eq). On laisse sous agitation et courant d'azote à température ambiante pendant environ 3 heures. On chauffe à environ 50°C pendant environ une heure. On sèche et reprend le résidu huileux par 5 cm³ d'eau distillée, puis ajoute 0,44 cm³ d'acide chlorhydrique 2N (2 éq). On laisse sous agitation pendant environ 30 mn, puis filtre, lave à l'eau distillée, et sèche. On obtient 220 mg de produit attendu (poudre blanche). Point de fusion 178°C.

| Microanalyse : C₂₇H₃₃N₅O₄S₂ = 555, 723 | | | | |
|---|---|---|---|---|
| | C | H | N | S |
| % calculés | 58,35 | 5,98 | 12,60 | 11,53 |
| % trouvés | 58,0 | 6,0 | 12,2 | 11,3 |

### EXEMPLE 8 : 4'-[(2-butyl) 6,7-dihydro-4-oxide 7-oxo-thiopyrano[2,3-d] imidazol-1-(5H)-yl) méthyl] N-[(diméthylamino) méthylène) (1,1'-biphényl) 2-sulfonamide,

On introduit 690 mg du produit de l'exemple 1 (1,35 mmoles), 6,9 cm³ de chlorure de méthylène. On introduit à 0°C une solution d'acide métachloroperbenzoique, 326 mg d'acide à 70 % (1,32 mmoles 1 éq) en solution dans 1,6 cm³ de chlorure de méthylène. On laisse 4 heures sous agitation et revenir à température ambiante. On traite la réaction en versant dans 30 cm³ d'eau plus 5 cm³ de solution de bicarbonate de sodium saturée. On extrait avec 3 fois 50 cm³ de chlorure de méthylène, filtre et sèche. On obtient 950 mg de produit brut que l'on purifie par chromatographie.

Les 950 mg de produit brut sont déposés en solution dans un volume de l'éluant chlorure de méthylène/méthanol (97-3) sur une colonne de silice. On élue, isole la fraction contenant le produit attendu et sèche. On obtient 570 mg de produit (poudre blanche).

### ANALYSES :

### Spectre IR, CHCl₃

>=O 1676, 1628 cm⁻¹
aromatique, hétéroatome 1516 cm⁻¹
>S --> O 1042 cm⁻¹.

### EXEMPLE 9 : 4'-[(2-butyl-7-oxo-thiopyrano [2,3-d] imidazol-1-(7H)-yl) méthyl] N-[(diméthylamino) méthylène) (1,1' biphényl) 2-sulfonamide

On introduit 500 mg du produit de l'exemple 8 (0,949 mmole), 5 cm³ de CH₂Cl₂, 1,5 cm³ d'anhydride acétique (3 vol), 0,15 cm³ d'acide méthane sulfonique (0,3 vol). On laisse à environ 40°C, pendant 15 heures, sous agitation. On traite en versant dans 20 cm³ d'eau, on amène à pH ∼ 6 par addition de solution saturée de bicarbonate de sodium. On extrait avec 3 x 50 cm³ de CH₂Cl₂, filtre et sèche. On obtient 500 mg de produit brut que l'on purifie par chromatographie. Les 500 mg de produit brut sont déposés sur une colonne de silice dans l'éluant CH₂Cl₂/MeOH (95-5). On élue et on isole la fraction contenant le produit attendu, sèche et obtient 394 mg de produit attendu.

### ANALYSES :

### Spectre IR, CHCl₃

>C=O, C=C, C=N 1628, 1606 cm⁻¹
Système conjugué + aromatique 1515 cm⁻¹.

### EXEMPLE 10 : 4'-[(2-butyl)-7-oxo-thiopyrano [2,3-d] imidazol-1-(7H)-yl) méthyl] (1,1'-biphényl) 2-sulfonamide

On introduit 390 mg du produit de l'exemple 9 (0,77 mmole), 4 cm³ d'éthanol (10 vol), 1,2 cm³ d'acide chlorhydrique concentré (3 vol). On porte environ 2 heures au reflux. On amène à sec, reprend par 10 cm³ d'eau, amène à pH basique par addition de 4 à 5 cm³ de soude concentrée. On extrait avec 3 x 50 cm³ d'AcOEt, filtre et sèche. On obtient 320 mg de produit brut que l'on purifie par chromatographie.

Les 320 mg de produit brut sont déposés en solution dans 1 volume de l'éluant CH₂Cl₂/AcOEt (50-50) sur une colonne de silice. On élue et on isole la fraction contenant le produit attendu, sèche et obtient 260 mg de produit attendu (poudre blanche).

### ANALYSES :

### Spectre IR, CHCl₃

Région OH/NH 3320, 3215 cm⁻¹
+ absorption générale
>=O 1609 cm⁻¹
C=C, aromatique, NH₂ déf. 1563, 1519, 1504 cm¹.

### EXEMPLE 11 : 4'-[(2-butyl) 7-oxo thiopyrano [2,3-d] imidazol 1-(7H)-yl) méthyl] N-((propylamino) carbonyl) (1,1'-biphényl) 2-sulfonamide

On introduit 250 mg du produit A de l'exemple 10, 3,75 cm³ de diméthoxy éthane (15 vol), puis 92 mg de carbonate de potassium (1,2 eq 0,65 mmoles). Puis 0,072 cm³ de propylisocyanate (1,4 eq 0,77 mmoles). On porte à environ 80°C pendant environ 2 heures. On verse dans 30 cm³ d'eau, plus 10 cm³ de solution saturée de chlorure d'ammonium. On extrait avec 3 fois 50 cm³ de chlorure de méthylène, filtre et sèche. On obtient 300 mg de produit brut que l'on purifie par chromatographie. Les 300 mg de produit brut sont déposés en solution dans l'éluant CH₂Cl₂/AcOEt (50-50) sur une colonne de silice. On élue, isole la fraction contenant le produit attendu et sèche. On obtient 285 mg de produit que l'on soumet à une 2ème purification. On introduit 285 mg de produit obtenu, 2,8 cm³ d'eau déminéralisée (10 vol), 0,529 cm³ de soude 2N, puis on précipite le produit attendu par addition de 0,529 cm³ d'HCl 2N. On laisse pendant environ 1/2 heure, filtre, lave le produit avec de l'eau déminéralisée et sèche. On obtient 245 mg de produit attendu. Point de fusion 162°C.

### ANALYSES :

### Spectre IR, CHCl₃

NH 3400 (ep) 3369 cm⁻¹ max.
>=O 1714, 1601 cm⁻¹
Système conjugué, aromatique, amide 1565, 1542, 1501 cm⁻¹.

| Microanalyse : C₂₇H₃₀N₄O₄S₂, M = 538,69, F = 162°C | | | | |
|---|---|---|---|---|
| | C | H | N | S |
| % calculés | 60,2 | 5,6 | 10,4 | 11,9 |
| % trouvés | 59,9 | 5,5 | 10,5 | 11,8 |

### EXEMPLE 12 : 4'-[(2-butyl) 6,7-dihydro 6-fluoro 7-oxo thiopyrano [2,3-d] imidazol-1-(5H)-yl) méthyl] N-((diméthylamino) méthylène) (1,1'-biphényl) 2-sulfonamide

### Stade A : 2-butyl 5,6-dihydro 1-[(2-(triméthylsilyl) éthoxy) méthyl) thiopyrano (2,3-d) imidazol 7(1H)-one.

On ajoute à température ambiante 0,99 ml de diisopropyléthylamine à 1 g de produit obtenu au stade 5 de l'exemple 1 dans 10 ml de dichlorométhane. On refroidit à 0°C, ajoute 1 ml de chlorure de 2-(triméthylsilyl) éthoxyméthyle, laisse revenir à température ambiante et agite pendant 2 heures. On verse dans l'eau, extrait au dichlorométhane, sèche et élimine le solvant sous pression réduite. Après chromatographie du résidu sur silice (éluant CH₂Cl₂-AcOEt 95-5), on obtient 1 g de produit attendu.

### ANALYSES :

### Spectre IR, CHCl₃

absence de =C-NH-
carbonyle : 1643 cm⁻¹
silyle : 840 cm⁻¹.

### Stade B : 2-butyl 5,6-dihydro 6-fluoro 1-[(2-(triméthylsilyl) éthoxy) méthyl) thiopyrano (2,3-d) imidazol 7(1H)-one.

On refroidit à -70°C, 1,76 g de produit obtenu comme au stade A dans 8,75 ml de tétrahydrofuranne puis ajoute 5,68 ml de bis triméthylsilylamine de lithium en solution molaire dans le tétrahydrofuranne. On agite 15 minutes à -70°C, ajoute 1,05 ml de trifluorométhane sulfonate de triméthylsilyle, agite 30 minutes à -70°C, ajoute 2,5 ml d'acétonitrile puis 2,1 g de 1-chlorométhyl 4-fluoro 1,4-diazonia bicyclo (2,2,2)-octane bis tétrafluoroborate. On maintient sous agitation à -70°C pendant 15 minutes, laisse revenir à 0°C sous agitation et atmosphère inerte. On verse dans l'eau glacée, ajoute 20 ml d'une solution aqueuse saturée en chlorure d'ammonium, extrait à l'acétate d'éthyle, sèche, élimine le solvant sous pression réduite, chromatographie le résidu sur silice (éluant : CH₂Cl₂-AcOEt 95-5) et obtient 305 mg de produit attendu.
rf = 0, 3 (CH₂Cl₂-AcOEt 95-5).

### Stade C : 2-butyl 5,6-dihydro 6-fluoro-thiopyrano (2,3-d) imidazol-7 (1H) -one.

On chauffe à 40°C pendant 12 heures 305 mg du produit obtenu au stade B et 1,75 ml d'acide trifluoroacétique dans 6,1 ml de dichlorométhane. On verse dans l'eau, ajoute du bicarbonate de sodium jusqu'à pH 6-7, extrait au chlorure de méthylène, élimine le solvant sous pression réduite, et obtient 180 mg de produit attendu.

### ANALYSES :

| Spectre IR, CHCl₃ | |
|---|---|
| C=O : | 1654 cm⁻¹ |
| C=C | 1537 cm⁻¹ |
| C=N | 1502 cm⁻¹. |

### Stade D : 4'-[(2-butyl) 6,7-dihydro 6-fluoro 7-oxo thiopyrano [2,3-d] imidazol-1-(5H)-yl) méthyl] N-((diméthylamino) méthylène) (1,1'-biphényl) 2-sulfonamide

On opère comme au stade D de l'exemple 1 en utilisant 170 mg de produit obtenu au stade C ci-dessus dans 2,55 ml de diméthylformamide, 125 mg de carbonate de potassium et 425 mg de réactif bromobiphényle. On obtient après chromatographie sur silice (éluant : CH₂Cl₂-AcOEt 50-50) 275 mg de produit attendu.

### ANALYSES :

| Spectre IR, CHCl₃ | |
|---|---|
| C=O | 1661 cm⁻¹ |
| N=CH-N< | 1630 cm⁻¹ |

### EXEMPLE 13 : 4'-[(2-butyl) 6,7-dihydro 6-fluoro 7-oxo thiopyrano [2,3-d] imidazol-1-(5H)-yl) méthyl] (1,1'-biphényl) 2-sulfonamide

On opère comme à l'exemple 2 en utilisant au départ, 330 mg de produit obtenu comme à l'exemple 12, 3,3 ml d'éthanol et 1 ml d'acide chlorhydrique concentré. On obtient 285 mg de produit brut que l'on utilise tel quel à l'exemple suivant. F = 208°C.

### ANALYSES :

### Spectre IR, CHCl₃

Absence de N-CH-N<
=O : 1680, 1671 cm⁻¹.
aromatique, hétéroaromatique, NH₂ def. : 1592, 1500 cm⁻¹.

### EXEMPLE 14 : 4'-[(2-butyl) 6,7-dihydro 6-fluoro 7-oxo thiopyrano [2,3-d] imidazol-1-(5H)-yl) méthyl] N-((propylamino) carbonyl) (1,1'-biphényl) 2-sulfonamide

On opère comme à l'exemple 3 en utilisant au départ 275 mg de produit obtenu à l'exemple 13, 2,75 ml de diméthoxyéthane, 100 mg de carbonate de potassium et 0,065 ml de propylisocyanate. Après extraction à l'acétate d'éthyle et chromatographie sur silice (éluant : dichlorométhane-acétate d'éthyle 7-3 puis 5-5), on obtient 183 mg de produit attendu que l'on reprend dans 3 ml d'eau, ajoute 0,28 ml de soude 2N, filtre, ajoute 0,28 ml d'acide chlorhydrique, agite 1 heure, filtre, lave à l'eau, sèche sous pression réduite à 50°C et récupère 100 mg de produit pur. F = 188°C.

### ANALYSES :

| Spectre IR, CHCl₃ | |
|---|---|
| =C-NH | 3400, 3371 cm⁻¹ |
| C=O | 1716, 1662 cm⁻¹. |
| amide II, système conj. | 1540 cm⁻¹. |

### EXEMPLE 15 : 4'-[(2-butyl) 7-chloro 1,5-dihydro 6-formyl thiopyrano [2,3-d] imidazol-1-yl) méthyl] N-((diméthylamino) méthylène) (1,1'-biphényl) 2-sulfonamide

On refroidit à 4°C, 50 ml de trichloréthylène et 0,61 ml de diméthylformamide, ajoute 0,37 ml de chlorure de phosphoryl, laisse revenir à température ambiante, ajoute 1 g du produit préparé au stade 6 de l'exemple 1 en solution dans 3 ml de diméthylformamide. On chauffe le milieu réactionnel 3 heures à 95° ± 5°C, laisse revenir à température ambiante, verse dans une solution aqueuse d'acétate de sodium et extrait au chlorure de méthylène. On élimine le solvant sous pression réduite, chromatographie le résidu sur silice (éluant : flugène-AcOEt 3-7) et obtient 624 mg de produit attendu que l'on recristallise dans l'acétate d'éthyle. F = 160°C.

### ANALYSES :

| Spectre IR, CHCl₃ | |
|---|---|
| C=O | 1648 cm⁻¹. |
| N=C-N | 1628 cm⁻¹ |
| aromatiques | 1592, 1551, 1516, 1487 cm⁻¹. |

### EXEMPLE 16 : 1-[(2'-(aminosulfonyl) (1,1'-biphényl) 4-yl) méthyl) 2-butyl 1,5-dihydro thiopyrano [2,3-d] imidazol 6-carboxylate d'éthyle.

On chauffe 4 heures et demie au reflux, 100,1 mg de produit obtenu à l'exemple 15, 2 ml d'éthanol et 0,1 ml d'acide chlorhydrique concentré. On refroidit à température ambiante, verse dans 20 ml d'eau, neutralise à l'aide de soude 2N, extrait au dichlorométhane, lave à l'eau, sèche, élimine le solvant sous pression réduite et obtient après chromatographie sur silice (éluant : AcOEt-cyclohexane 5-5), 34 mg de produit attendu.

### ANALYSES :

| Spectre IR, CHCl₃ absence de -N=CH-N- (CH₃)₂ | |
|---|---|
| NH₂ | 3443, 3340 cm⁻¹ |
| C=O | 1655 cm⁻¹. |
| C=C | 1585 cm⁻¹ |

### EXEMPLE 17 : 2-butyl 7-chloro 1,5-dihydro 1-[[2'-[[[(diméthylamino) méthylène] amino] sulfonyl] (1,1'-biphényl) 4-yl] méthyl] thiopyrano [2,3-d] imidazol 6-carboxylate de méthyle.

On agite 2 heures à température ambiante 285 mg de produit obtenu comme à l'exemple 15, 6 ml de méthanol, 900 mg d'oxyde de manganèse, 127 mg de cyanure de sodium et 0,05 ml d'acide acétique. On dilue avec 10 ml d'acétate d'éthyle, filtre, lave à l'acétate d'éthyle, élimine le solvant sous pression réduite, chromatographie le résidu sur silice (éluant : flugène-AcOEt 3-7) et obtient 220 mg de produit attendu.

### ANALYSES :

| Spectre IR, CHCl₃ | |
|---|---|
| C=O | 1712 cm⁻¹. |
| N=C-N | 1627 cm⁻¹ |
| syst. conj. + aromatiques | 1554, 1492 cm⁻¹. |

### EXEMPLE 18 : 1'-[(2'-(aminosulfonyl) (1,1'-biphényl) 4-yl) méthyl) 2-butyl 7-chloro 1,5-dihydro thiopyrano [2,3-d] imidazol 6-carboxylate de méthyle.

On chauffe 7 heures et demie au reflux, 194 mg du produit obtenu à l'exemple 17, 4 ml de méthanol et 0,2 ml d'acide chlorhydrique concentré. On verse dans l'eau, neutralise à la soude 2N, extrait au dichlorométhane, lave à l'eau, sèche et élimine le solvant sous pression réduite. Après chromatographie sur silice (éluant : cyclohexanne-AcOEt 6-4), on obtient 100 mg de produit attendu.

### ANALYSES :

| Spectre IR, CHCl₃ | |
|---|---|
| NH₂ | : 3444 cm⁻¹ |
| C=O complexe | : 1712 cm⁻¹ (max). |
| syst. conj. | |
| + aromatiques | 1600, 1590, 1554, 1517, 1494 cm⁻¹. |
| + NH₂ | |

### EXEMPLE 19 : 2-butyl 7-chloro 1,5-dihydro 1-[[2'-[[[(propylamino) carbonyl] amino] sulfonyl] (1,1'-biphényl) 4-yl] méthyl] thiopyrano [2,3-d] imidazol 6-carboxylate de méthyle.

On opère comme à l'exemple 3 en utilisant au départ 91 mg de produit obtenu à l'exemple 18 dans 0,9 ml de diméthoxyéthane. 60 mg de carbonate de potassium et 0,022 ml de propylisocyanate. On obtient 84 mg de produit attendu.

### ANALYSES :

| Spectre IR, CHCl₃ | |
|---|---|
| =C-NH | : 3404 cm⁻¹ |
| C=O | : 1715 cm⁻¹ (max) |
| syst. conj. | |
| + amide II | 1600, 1590, 1543, 1494 cm⁻¹. |
| + aromatique | |

### EXEMPLE 20 : acide 2-butyl 7-chloro 1,5-dihydro 1-[[2'[[[(propylamino) carbonyl] amino] sulfonyl] (1,1'-biphényl)-4-yl] méthyl] thiopyrano [2,3-d] imidazol 6-carboxylique.

On agite 24 heures à température ambiante 79 mg de produit obtenu à l'exemple 19, 1,1 ml d'éthanol et 0,26 ml de soude 2N. On élimine les solvants sous pression réduite, reprend dans 2 ml d'eau, neutralise avec de l'acide chlorhydrique 2N, agite 2 heures à température ambiante, filtre, lave à l'eau, sèche sous pression réduite et recueille 69,7 mg de produit attendu. F ≈ 200°C.

### ANALYSES

| Spectre IR, Nujol Absorption complexe OH/NH | |
|---|---|
| C=O | : 1718 (ep), 1688 (ep), 1669 cm⁻¹ (max) . |
| C=C | |
| + aromatiques | 1594, 1556, 1496 cm⁻¹. |
| + Amide II | |

### EXEMPLE 21 : (2-butyl 1-[[2'-[[[(diméthylamino) méthylène] amino] sulfonyl] (1,1'-biphényl) 4-yl] méthyl] 7-hydroxy 1,5,6,7-tétrahydrothiopyrano [2,3-d] imidazol-7-yl] acétate d'éthyle.

### 1) Préparation du réactif organozincique BrZn-CH₂-CO₂Et.

On mélange sous atmosphère inerte 3,25 g de zinc et 2 ml de tétrahydrofuranne, ajoute 0,2 ml de 1,2-dibromoéthane et chauffe au reflux pendant 5 minutes, ajoute en 1 h en maintenant la température à 40°-50°C 4,44 ml de bromoacétate d'éthyle dissous dans 40 ml de tétrahydrofuranne. On agite 30 mn à température ambiante et utilise tel que. titre 0,4 M/l.

### 2) Condensation.

On introduit 25 ml de réactif préparé ci-dessus dans une solution comprenant 1 g de produit obtenu au stade 6 de l'exemple 1 dans 20 ml de tétrahydrofuranne sous atmosphère inerte. On agite 16 heures à température ambiante, verse dans une solution aqueuse saturée en chlorure d'ammonium, extrait à l'acétate d'éthyle, lave à l'eau salée, sèche et évapore le solvant. On chromatographie le résidu sur silice (éluant : CH₂Cl₂-acétone 8-2) et obtient 224 mg de produit attendu.

### ANALYSES :

| Spectre IR, CHCl₃ | |
|---|---|
| Absorption OH | 3450 cm⁻¹ |
| C=O | 1713 cm⁻¹ |
| -N=CH-N | 1628 cm⁻¹ |

### EXEMPLE 22 : (2-butyl 1-[[2'-[[[(diméthylamino) méthylène] amino] sulfonyl] (1,1'-biphényl) 4-yl] méthyl] 1,5,6,7-tétrahydrothiopyrano [2,3-d] imidazol-7-ylidène] acétate d'éthyle.

On agite 2 heures au reflux 875 mg de produit obtenu comme à l'exemple 21 et 20 ml de toluène en présence de 14 mg d'acide paratoluènesulfonique. On évapore le solvant, chromatographie le résidu sur silice (éluant : CH₂Cl₂-AcOEt 5-5) et obtient 593 mg de produit attendu. F = 100°C.

### ANALYSES :

| Spectre IR, CHCl₃ Absence d'OH | |
|---|---|
| C=O | 1700 cm⁻¹ (ester conj.) |
| -N=CH-N | 1528 cm⁻¹ |
| C=C + aromatique | 1504 cm⁻¹ |

### EXEMPLE 23 : 1-[[2'-(aminosulfonyl) (1,1'-biphényl)-4-yl] méthyl] 2-butyl 1,5,6,7-tétrahydrothiopyrano [2,3-d] imidazol 7-ylidène-acétate d'éthyle.

On agite 6 heures au reflux 570 mg de produit obtenu à l'exemple 22 dans 5,7 ml d'éthanol et 0,57 ml d'acide chlorhydrique 2N. On abandonne 16 heures à température ambiante, verse dans l'eau, neutralise à l'aide de soude 2N, extrait au dichlorométhane, lave à l'eau, sèche et évapore le solvant. Après chromatographie sur silice (éluant : CH₂Cl₂-AcOEt 8-2), on obtient 225 mg de produit attendu.

### ANALYSES :

| Spectre IR, CHCl₃ Absence de N=CH-N | |
|---|---|
| NH₂ | 3442, 3344 cm⁻¹ |
| C=O | 1699 cm⁻¹ |
| C=C + aromatique | 1600, 1540, 1503 cm⁻¹ |

### EXEMPLE 24 : (2-butyl 1-[[2'-[[[(propylamino) carbonyl] amino] sulfonyl] (1,1'-biphényl) 4-yl] méthyl] 1,5,6,7-tétrahydrothiopyrano [2,3-d] imidazol-7-ylidène] acétate d'éthyle.

On opère comme à l'exemple 3 en utilisant au départ 210 mg de produit obtenu à l'exemple 23, 2,1 ml de diméthoxyéthane, 110 mg de carbonate de potassium et 0,056 ml de propylisocyanate. On obtient 147 mg de produit attendu. F = 170°C.

### ANALYSES :

| Spectre IR, CHCl₃ | |
|---|---|
| -C=NH | : 3400, 3380 cm⁻¹ |
| C=O | : 1715, 1654 cm⁻¹ |
| Syst. conj. | |
| + aromatique | 1595, 1540, 1503 cm⁻¹ |
| + amide II | |

### EXEMPLE 25 : acide 2-(2-butyl 1-[[2'[[[(propylamino) carbonyl] amino] sulfonyl] (1,1'-biphényl)-4-yl] méthyl] 1,5,6,7-tétrahydrothiopyrano [2,3-d] imidazol 7-ylidène] acétique.

On opère comme à l'exemple 20 en utilisant au départ 135 mg de produit obtenu à 1'exemple 24, 2,7 ml d'éthanol et 0,44 ml de soude 2N. On obtient 112 mg de produit attendu. F = 130°C.

### ANALYSES :

| Spectre IR, CHCl₃ Absorption générale NH/OH | |
|---|---|
| C=O | : 1704, 1695 cm⁻¹ |
| Syst. conj. | |
| + aromatique | 1592, 1542, 1510 cm⁻¹ |
| + amide II | |

### EXEMPLE 26 : acide [[[1-[[2'-(aminosulfonyl] (1,1'-biphényl)-4-yl] méthyl] 2-butyl] 1,5,6,7-tétrahydrothiopyrano [2,3-d] imidazol 7-ylidène] amino] oxy] acétique.

On mélange 370 mg de produit obtenu comme à l'exemple 2 dans 3,7 ml d'éthanol et 3,7 ml de méthanol. On ajoute 400 mg d'acétate de sodium et 524 mg d'hémichlorure de carboxyméthylamine et chauffe 48 heures au reflux. On ajoute 400 mg d'acétate de sodium et 520 mg d'hémichlorure de carboxyméthylamine, poursuit le reflux pendant 24 heures, laisse refroidir le milieu réactionnel, verse dans l'eau, acidifie par addition d'acide chlorhydrique 2N, extrait à l'acétate d'éthyle, sèche, évapore le solvant sous pression réduite et obtient après chromatographie sur silice (éluant : CH₂Cl₂-MeOH 95-5) 120 mg de produit attendu.

### ANALYSES :

| Spectre IR, Nujol Absorption générale NH/OH | |
|---|---|
| C=O | : 1738, 1680 cm⁻¹ |
| Syst. conj. | |
| + aromatique | 1590, 1520 cm⁻¹ |
| + COO⁻ | |

### EXEMPLE 27 : acide 2-[[[2-butyl 1-[[2'-[[[[(propylamino) carbonyl] amino] sulfonyl] (1,1'-biphényl)-4-yl] méthyl] 1,5,6,7-tétrahydrothiopyrano [2,3-d] imidazol 7-ylidène] amino] oxy] acétique.

On opère comme à l'exemple 14 en utilisant au départ 165 mg de produit préparé comme à l'exemple 26, 2,4 ml de diméthoxyéthane, 95 mg de carbonate de potassium et 0,038 ml de propylisocyanate. On obtient après chromatographie sur silice (éluant : AcOEt-AcOH 99-1) 108 mg de produit puis après purification, 72 mg de produit attendu. F = 132-1340C.

### ANALYSES :

| Spectre IR, Nujol Absorption générale NH/OH | |
|---|---|
| C=O | 1710, 1670 cm⁻¹ |
| C=N, aromatique, hétérocycle, COO⁻ | 1600, 1545, 1520 cm⁻¹ |

### EXEMPLE 28 : 4'-[[2-butyl 7-hydroxy 7-méthyl 1,5,6,7-tétrahydrothiopyrano [2,3-d] imidazol 1-yl] méthyl] N-[(diméthylamino) méthylène] (1,1'biphényl) 2-sulfonamide.

On opère comme à l'exemple 21 en utilisant au départ 102 mg de produit préparé au stade 6 de l'exemple 1, 5 ml de tétrahydrofuranne et 0,333 ml d'une solution 3M de chlorure de méthyl magnésium dans le tétrahydrofuranne. On obtient 56 mg de produit attendu.

### ANALYSES :

| Spectre IR, CHCl₃ | |
|---|---|
| Absorption OH + Associé | 3590 cm⁻¹ |
| N=CH-N | 1627 cm⁻¹ |
| aromatique | 1590 cm⁻¹ |
| hétérocycle | 1540, 1512 cm⁻¹ |

### EXEMPLE 29 : 4'-[[2-butyl 6,7-dihydro 6-hydroxyméthyl 7-oxo thiopyrano [2,3-d] imidazol 1(5H)-yl] méthyl] N-[(diméthylamino) méthylène] (1,1'biphényl) 2-sulfonamide.

### 1) Préparation de l'organomagnésien.

On mélange sous atmosphère inerte 600 mg de magnésium en tournures, un peu de chlorure mercurique, agite 5 minutes, ajoute 2 ml de tétrahydrofuranne et 0,1 ml de chlorométhyl-benzyléther et chauffe à 40°-45°C. On refroidit à 0°/+5°C et ajoute en 1 heure 2,8 ml de chlorométhylbenzyléther et 20 ml de tétrahydrofuranne et agite 16 heures à 0°/+5°C. On obtient une solution 0,2 M/l utilisée telle quelle.

### 2) Condensation.

On refroidit sous atmosphère inerte à -30°C, 20 ml de la solution d'organomagnésien préparée ci-dessus et ajoute en 10 minuces,280 mg de produit préparé au stade 6 de l'exemple 1 dans 10 ml de tétrahydrofuranne. On agite 96 heures à 0°C, verse dans une solution aqueuse saturée en chlorure d'ammonium, extrait à l'acétate d'éthyle, lave à l'eau salée, sèche, évapore le solvant, chromatographie le résidu sur silice (éluant : CH₂Cl₂-acétone 8-2) et obtient 52 mg de produit attendu.

### ANALYSES :

| Spectre IR, CHCl₃ | |
|---|---|
| Absorption OH + associé | 3615 cm⁻¹ |
| N=CH-N | 1738, 1680 cm⁻¹ |

### EXEMPLE 30 :

En opérant comme indiqué dans les exemples précédents, on a préparé le produit suivant :
- acide 2-butyl 1-[[2'-[[(éthoxycarbonyl] amino] sulfonyl] (1,1'-biphényl)-4-yl] méthyl] 1,5,6,7-tétrahydrothiopyrano [2,3-d] imidazol 7-carboxylique.

En opérant comme indiqué dans les exemples précédents à partir du 2-butyl 1,5-dihydrothiopyrano [2,3-d] imidazol 7-carbonitrile, on a obtenu les produits correspondants aux exemples 1, 2,3, 4 comportant un radical carbonitrile en position 7.

### Préparation du 2-butyl 1,5-dihydrothiopyrano [2,3-d] imidazol 7-carbonitrile.

On agite à 60°C une suspension comprenant 300 mg de produit obtenu au stade 5 de l'exemple 1,30 ml de toluène, 0,38 ml de triméthylsilylcyanure et 32 mg d'iodure de zinc. Après 16 heures d'agitation à 60°C, on effectue l'addition d'une même quantité de réactif silylé et d'iodure de zinc puis de nouveau après 24 heures d'agitation à 60°C. On poursuit l'agitation à 60°C pendant 48 heures supplémentaires. On ajoute alors 5 ml de pyridine et 0,77 ml d'oxychlorure de phosphore et agite à 80°C pendant 4 heures. On refroidit le milieu réactionnel, le verse dans 50 ml d'eau, extrait à l'acétate d'éthyle, lave avec une solution salée, sèche, évapore le solvant sous pression réduite, chromatographie le résidu sur silice (éluant : dichlorométhane-acétate d'éthyle 7-3) et obtient 46 mg de produit attendu.
rf = 0,44 (CHCl₂-AcOEt 7-3).

### EXEMPLE 31 de composition pharmaceutique

On a préparé des comprimés répondant à la formule

| | |
|---|---|
| Produit de l'exemple 3 | 10 mg |
| Excipient pour un comprimé terminé à | 100 mg |
| (détail de l'excipient : lactose, talc, amidon, stéarate de magnésium) | |

### RESULTATS PHARMACOLOGIOUES

### 1 - Test sur le récepteur de l'angiotensine II

On utilise une préparation membranaire fraîche obtenue à partir de foie de rat. Le tissu est broyé au polytron dans un tampon Tris 50 mM pH 7,4, le broyage est suivi de 3 centrifugations à 30 000 g pendant 15 minutes avec reprises intermédiaires des culots dans le tampon Tris pH 7,4.

Les derniers culots sont remis en suspension dans un tampon d'incubation (Tris 20 mM, NaCl 135 mM, KCl 10 mM, glucose 5 mM, MgCl₂ 10 mM PMSF 0,3 mM, bacitracine 0,1 mM, BSA 0,2 %) .

On répartit des fractions aliquotées de 2 cm³ dans des tubes à hémolyse et ajoute de la ¹²⁵ I angiotensine II (25 000 DPM/tube) et le produit à étudier. Le produit est d'abord testé à 3 x 10⁻⁵M en triple. Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %).

La liaison non spécifique est déterminée par addition du produit de l'exemple 94 du brevet européen 0253310, à 10⁻⁵M (en triple). On incube à 25°C pendant 150 minutes, remet au bain-marie à 0°C, 5 minutes, filtre sous vide, rince au tampon Tris pH 7,4 et compte la radioactivité en présence du scintillant Triton.

Le résultat est exprimé directement en concentration inhibitrice 50 % (CI₅₀), c'est-à-dire en concentration de produit étudié, exprimée en nM, nécessaire pour déplacer 50 % de la radioactivité spécifique fixée sur le récepteur étudié.

### Résultats :

| Produit de l'exemple | CI₅₀ en nanomoles |
|---|---|
| 3. | 1,3 |
| 4. | 0,2 |
| 7 | 5,3 |

### 2 - Test d'activité antagoniste de l'angiotensine II chez le rat démédullé

Des rats mâles Sprague-Dawley (250 à 350 g) sont anesthésiés par une injection intra-péritonéale de pentobarbital sodique (60 mg/kg). La pression artérielle diastolique est enregistrée grâce à un cathéter (PE50) hépariné introduit dans la carotide gauche de l'animal, et relié à un calculateur de pression (Gould, Pressure Processor) par l'intermédiaire d'un capteur de pression Gould.

Un cathéter est introduit dans la jugulaire droite de l'animal pour l'injection des molécules à étudier.

L'animal est placé sous respiration assistée. Une section bilatérale des nerfs vagues est effectuée. Le rat est alors démédullé.

Après une période de stabilisation suffisante, l'étude de l'antagonisme des composés vis-à-vis de l'angiotensine II (Hypertensine Ciba) est abordée de la façon suivante.
1 - Trois injections consécutives d'angiotensine II (0,75 microgrammes/kg) espacées de 15 minutes permettent d'obtenir une réponse pressive reproductible et stable.
2 - Tout en gardant une périodicité de 15 minutes pour l'administration d'angiotensine II, les molécules à étudier sont injectées 5 minutes avant l'angiotensine II.

Les effets presseurs de l'angiotensine II en présence de l'antagoniste sont exprimés en pourcentage des effets presseurs de l'angiotensine II administrée seule. La dose inhibitrice à 50 % de l'effet étudié est ainsi déterminée

Chaque animal est considéré comme son propre témoin.

### Résultats :

| Produit de l'exemple | Dose inhibitrice à 50 % (mg/kg) (voie veineuse) |
|---|---|
| 3 | 0,08 |
| 4 | 0,09 |

| | |
|---|---|
| C=O complexe | : 1712 cm⁻¹ (max). |
| syst. conj. | |
| + aromatiques | 1600, 1590, 1554, 1517, 1494 cm⁻¹. |
| + NH₂ | |

### EXEMPLE 19 : 2-butyl 7-chloro 1,5-dihydro 1-[[2'-[[[(propylamino) carbonyl] amino] sulfonyl] (1,1'-biphényl) 4-yl] méthyl] thiopyrano [2,3-d] imidazol 6-carboxylate de méthyle.

On opère comme à l'exemple 3 en utilisant au départ 91 mg de produit obtenu à l'exemple 18 dans 0,9 ml de diméthoxyéthane, 60 mg de carbonate de potassium et 0,022 ml de propylisocyanate. On obtient 84 mg de produit attendu.

### ANALYSES :

| Spectre IR, CHCl₃ | |
|---|---|
| =C-NH | : 3404 cm⁻¹ |
| C=O | : 1715 cm⁻¹ (max) |
| syst. conj. | |
| + amide II | 1600, 1590, 1543, 1494 cm⁻¹. |
| + aromatique | |

### EXEMPLE 20 : acide 2-butyl 7-chloro 1,5-dihydro 1-[[2'[[[(propylamino) carbonyl] amino] sulfonyl] (1,1'-biphényl)-4-yl] méthyl] thiopyrano [2,3-d] imidazol 6-carboxylique.

On agite 24 heures à température ambiante 79 mg de produit obtenu à l'exemple 19, 1,1 ml d'éthanol et 0,26 ml de soude 2N. On élimine les solvants sous pression réduite, reprend dans 2 ml d'eau, neutralise avec de l'acide chlorhydrique 2N, agite 2 heures à température ambiante, filtre, lave à l'eau, sèche sous pression réduite et recueille 69,7 mg de produit attendu. F ≈ 200°C.

### ANALYSES :

| Spectre IR, Nujol Absorption complexe OH/NH | |
|---|---|
| C=O | : 1718 (ep), 1688 (ep), 1669 cm⁻¹ (max). |
| C=C | |
| + aromatiques | 1594, 1556, 1496 cm⁻¹. |
| + Amide II | |

### EXEMPLE 21 : (2-butyl 1-[[2'-[[[(diméthylamino) méthylène] amino] sulfonyl] (1,1'-biphényl) 4-yl] méthyl] 7-hydroxy 1,5,6,7-tétrahydrothiopyrano [2,3-d] imidazol-7-yl] acétate d'éthyle.

### 1) Préparation du réactif organizincique BrZn-CH₂-CO₂Et.

On mélange sous atmosphère inerte 3,25 g de zinc et 2 ml de tétrahydrofuranne, ajoute 0,2 ml de 1,2-dibromoéthane et chauffe au reflux pendant 5 minutes, ajoute en 1 heure en maintemant la température à 40°-50°C de bromoacétate d'éthyle dissous dans 40 ml de tétrahydrofuranne. On agite 30 minutes a température ambiante et utilise tel que. titre 0,4 M/l.

### 2) Condensation.

On introduit 25 ml de réactif préparé ci-dessus dans une solution comprenant 1 g de produit obtenu au stade 6 de l'exemple 1 dans 20 ml de tétrahydrofuranne sous atmosphère inerte. On agite 16 heures à température ambiante, verse dans une solution aqueuse saturée en chlorure d'ammonium, extrait à l'acétate d'éthyle, lave à l'eau salée, sèche et évapore le solvant. On chromatographie le résidu sur silice (éluant : CH₂Cl₂-acétone 8-2) et obtient 224 mg de produit attendu.

### ANALYSES :

| Spectre IR, CHCl₃ | |
|---|---|
| Absorption OH | 3450 cm⁻¹ |
| C=O | 1713 cm⁻¹ |
| -N=CH-N | 1628 cm⁻¹ |

### EXEMPLE 22 : (2-butyl 1-[[2'-[[[(diméthylamino) méthylène] amino] sulfonyl] (1,1'-biphényl) 4-yl] méthyl] 1,5,6,7-tétrahydrothiopyrano [2,3-d] imidazol-7-ylidène] acétate d'éthyle.

On agite 2 heures au reflux 875 mg de produit obtenu comme à l'exemple 21 et 20 ml de toluène en présence de 14 mg d'acide paratoluènesulfonique. On évapore le solvant, chromatographie le résidu sur silice (éluant : CH₂Cl₂-AcOEt 5-5) et obtient 593 mg de produit attendu. F = 100°C.

### ANALYSES :

| Spectre IR, CHCl₃ Absence d'OH | |
|---|---|
| C=O | 1700 cm⁻¹ (ester conj.) |
| -N=CH-N | 1628 cm⁻¹ |
| C=C + aromatique | 1504 cm⁻¹ |

### EXEMPLE 23 : 1-[[2'-(aminosulfonyl) (1,1'-biphényl)-4-yl] méthyl] 2-butyl 1,5,6,7-tétrahydrothiopyrano [2,3-d] imidazol 7-ylidène-acétate d'éthyle.

On agite 6 heures au reflux 570 mg de produit obtenu à l'exemple 22 dans 5,7 ml d'éthanol et 0,57 ml d'acide chlorhydrique 2N. On abandonne 16 heures à température ambiante, verse dans l'eau, neutralise à l'aide de soude 2N, extrait au dichlorométhane, lave à l'eau, sèche et évapore le solvant. Après chromatographie sur silice (éluant : CH₂Cl₂-AcOEt 8-2), on obtient 225 mg de produit attendu.

### ANALYSES :

| Spectre IR, CHCl₃ Absence de N=CH-N | |
|---|---|
| NH₂ | 3442, 3344 cm⁻¹ |
| C=O | 1699 cm⁻¹ |
| C=C + aromatique | 1600, 1540, 1503 cm⁻¹ |

### EXEMPLE 24 : (2-butyl 1-[[2'-[[[(propylamino) carbonyl] amino] sulfonyl] (1,1'-biphényl) 4-yl] méthyl] 1,5,6,7-tétrahydrothiopyrano [2,3-d] imidazol-7-ylidène] acétate d'éthyle.

On opère comme à l'exemple 3 en utilisant au départ 210 mg de produit obtenu à l'exemple 23, 2,1 ml de diméthoxyéthane, 110 mg de carbonate de potassium et 0,056 ml de propylisocyanate. On obtient 147 mg de produit attendu. F = 170°C.

### ANALYSES :

| Spectre IR, CHCl₃ | |
|---|---|
| -C=NH | : 3400, 3380 cm⁻¹ |
| C=O | : 1715, 1654 cm⁻¹ |
| Syst. conj. | |
| + aromatique | 1595, 1540, 1503 cm⁻¹ |
| + amide II | |

### EXEMPLE 25 : acide 2-(2-butyl 1-[[2'[[[(propylamino) carbonyl] amino] sulfonyl] (1,1'-biphényl)-4-yl] méthyl] 1,5,6,7-tétrahydrothiopyrano [2,3-d] imidazol 7-ylidène] acétique.

On opère comme à l'exemple 20 en utilisant au départ 135 mg de produit obtenu à l'exemple 24, 2,7 ml d'éthanol et 0,44 ml de soude 2N. On obtient 112 mg de produit attendu. F = 130°C.

### ANALYSES :

| Spectre IR, CHCl₃ Absorption générale NH/OH | |
|---|---|
| C=O | : 1704, 1695 cm⁻¹ |
| Syst. conj. | |
| + aromatique | 1592, 1542, 1510 cm⁻¹ |
| + amide II | |

### EXEMPLE 26 : acide [[[1-[[2'-(aminosulfonyl] (1,1'-biphényl)-4-yl] méthyl] 2-butyl] 1,5,6,7-tétrahydrothiopyrano [2,3-d] imidazol 7-ylidène] amino] oxy] acétique.

On mélange 370 mg de produit obtenu comme à l'exemple 2 dans 3,7 ml d'éthanol et 3,7 ml de méthanol. On ajoute 400 mg d'acétate de sodium et 524 mg d'hémichlorure de carboxy-méthylamine et chauffe 48 heures au reflux. On ajoute 400 mg d'acétate de sodium et 520 mg d'hémichlorure de carboxy-méthylamine, poursuit le reflux pendant 24 heures, laisse refroidir le milieu réactionnel, verse dans l'eau, acidifie par addition d'acide chlorhydrique 2N, extrait à l'acétate d'éthyle, sèche, évapore le solvant sous pression réduite et obtient après chromatographie sur silice (éluant : CH₂Cl₂-MeOH 95-5) 120 mg de produit attendu.

### ANALYSES :

| Spectre IR, Nujol Absorption générale NH/OH | |
|---|---|
| C=O | : 1738, 1680 cm⁻¹ |
| Syst. conj. | |
| + aromatique | 1590, 1520 cm⁻¹ |
| + COO⁻ | |

### EXEMPLE 27 : acide 2-[[[2-butyl 1-[[2'-[[[[(propylamino) carbonyl] amino] sulfonyl] (1,1'-biphényl)-4-yl) méthyl] 1,5,6,7-tétrahydrothiopyrano [2,3-d] imidazol 7-ylidène] amino] oxy] acétique.

On opère comme à l'exemple 14 en utilisant au départ 165 mg de produit préparé comme à l'exemple 26, 2,4 ml de diméthoxyéthane, 95 mg de carbonate de potassium et 0,038 ml de propylisocyanate. On obtient après chromatographie sur silice (éluant : AcOEt-AcOH 99-1) 108 mg de produit puis après purification, 72 mg de produit attendu. F = 132-134°C.

### ANALYSES :

| Spectre IR, Nujol Absorption générale NH/OH | |
|---|---|
| C=O | 1710, 1670 cm⁻¹ |
| C=N, aromatique, hétérocycle, COO⁻ | 1600, 1545, 1520 cm⁻¹ |

### EXEMPLE 28 : 4'-[[2-butyl 7-hydroxy 7-méthyl 1,5,6,7-tétrahydrothiopyrano [2,3-d] imidazol 1-yl] méthyl] N-[(diméthylamino) méthylène] (1,1'biphényl) 2-sulfonamide.

On opère comme à l'exemple 21 en utilisant au départ 102 mg de produit préparé au stade 6 de l'exemple 1, 5 ml de tétrahydrofuranne et 0,333 ml d'une solution 3M de chlorure de méthyl magnésium dans le tétrahydrofuranne. On obtient 56 mg de produit attendu.

### ANALYSES :

| Spectre IR, CHCl₃ | |
|---|---|
| Absorption OH + Associé | 3590 cm⁻¹ |
| N=CH-N | 1627 cm⁻¹ |
| aromatique | 1590 cm⁻¹ |
| hétérocycle | 1540, 1512 cm⁻¹ |

### EXEMPLE 29 : 4'-[[2-butyl 6,7-dihydro 6-hydroxyméthyl 7-oxo thiopyrano [2,3-d] imidazol 1(5H)-yl] méthyl] N-[(diméthylamino) méthylène] (1,1'biphényl) 2-sulfonamide.

### 1) Préparation de l'organomagnésien.

On mélange sous atmosphère inerte 600 mg de magnésium en tournures, un peu de chlorure mercurique, agite 5 minutes, ajoute 2 ml de tétrahydrofuranne et 0,1 ml de chlorométhylbenzyléther et chauffe à 40°-45°C. On refroidit à 0°/+5°C et ajoute en 1 heure 2,8 ml de chlorométhylbenzyléther et 20 ml de tétrahydrofuranne et agite 16 heures à 0°/+5°C. On obtient une solution 0,2 M/l utilisée telle quelle.

### 2) Condensation.

On refroidit sous atmosphère inerte à -30°C, 20 ml de la solution d'organomagnésien préparée ci-dessus et ajoute en 10 minutes,280 mg de produit préparé au stade 6 de l'exemple 1 dans 10 ml de tétrahydrofuranne. On agite 96 heures à 0°C, verse dans une solution aqueuse saturée en chlorure d'ammonium, extrait à l'acétate d'éthyle, lave à l'eau salée, sèche, évapore le solvant, chromatographie le résidu sur silice (éluant : CH₂Cl₂-acétone 8-2) et obtient 52 mg de produit attendu.

### ANALYSES :

| Spectre IR, CHCl₃ | |
|---|---|
| Absorption OH + associé | 3615 cm⁻¹ |
| N=CH-N | 1738, 1680 cm⁻¹ |

### EXEMPLE 30 :

En opérant comme indiqué dans les exemples précédents, on a préparé le produit suivant :
- acide 2-butyl 1-[[2'-[[(éthoxycarbonyl] amino] sulfonyl] (1,1'-biphényl)-4-yl] méthyl] 1,5,6,7-tétrahydrothiopyrano [2,3-d] imidazol 7-carboxylique.

En opérant comme indiqué dans les exemples précédents à partir du 2-butyl 1,5-dihydrothiopyrano [2,3-d] imidazol 7-carbonitrile, on a obtenu les produits correspondants aux exemples 1, 2,3, 4 comportant un radical carbonitrile en position 7.

### Préparation du 2-butyl 1,5-dihydrothiopyrano [2,3-d] imidazol 7-carbonitrile.

On agite à 60°C une suspension comprenant 300 mg de produit obtenu au stade 5 de l'exemple 1,30 ml de toluène, 0,38 ml de triméthylsilylcyanure et 32 mg d'iodure de *zinc.* Après 16 heures d'agitation à 60°C, on effectue l'addition d'une même quantité de réactif silylé et d'iodure de zinc puis de nouveau après 24 heures d'agitation à 60°C. On poursuit l'agitation à 60°C pendant 48 heures supplémentaires. On ajoute alors 5 ml de pyridine et 0,77 ml d'oxychlorure de phosphore et agite à 80°C pendant 4 heures. On refroidit le milieu réactionnel, le verse dans 50 ml d'eau, extrait à l'acétate d'éthyle, lave avec une solution salée, sèche, évapore le solvant sous pression réduite, chromatographie le résidu sur silice (éluant : dichlorométhane-acétate d'éthyle 7-3) et obtient 46 mg de produit attendu.
rf = 0,44 (CHCl₂-AcOEt 7-3).

### EXEMPLE 31 de composition pharmaceutique

On a préparé des comprimés répondant à la formule suivante :

| | |
|---|---|
| Produit de l'exemple 3 | 10 mg |
| Excipient pour un comprimé terminé à | 100 mg |
| (détail de l'excipient : lactose, talc, amidon, stéarate de magnésium) | |

### RESULTATS PHARMACOLOGIOUES

### 1 - Test sur le récepteur de l'angiotensine II

On utilise une préparation membranaire fraîche obtenue à partir de foie de rat. Le tissu est broyé au polytron dans un tampon Tris 50 mM pH 7,4, le broyage est suivi de 3 centrifugations à 30 000 g pendant 15 minutes avec reprises intermédiaires des culots dans le tampon Tris pH 7,4.

Les derniers culots sont remis en suspension dans un tampon d'incubation (Tris 20 mM, NaCl 135 mM, KCl 10 mM, glucose 5 mM, MgCl₂ 10 mM PMSF 0,3 mM, bacitracine 0,1 mM, BSA 0,2 %).

On répartit des fractions aliquotées de 2 cm³ dans des tubes à hémolyse et ajoute de la ¹²⁵ I angiotensine II (25 000 DPM/tube) et le produit à étudier. Le produit est d'abord testé à 3 x 10⁻⁵M en triple. Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %).

La liaison non spécifique est déterminée par addition du produit de l'exemple 94 du brevet européen 0253310, à 10⁻⁵M (en triple). On incube à 25°C pendant 150 minutes, remet au bain-marie à 0°C, 5 minutes, filtre sous vide, rince au tampon Tris pH 7,4 et compte la radioactivité en présence du scintillant Triton.

Le résultat est exprimé directement en concentration inhibitrice 50 % (CI₅₀), c'est-à-dire en concentration de produit étudié, exprimée en nM, nécessaire pour déplacer 50 % de la radioactivité spécifique fixée sur le récepteur étudié.

### Résultats :

| Produit de l'exemple | CI₅₀ en nanomoles |
|---|---|
| 3 | 1,3 |
| 4 | 0,2 |
| 7 | 5,3 |

### 2 - Test d'activité antagoniste de l'angiotensine II chez le rat démédullé

Des rats mâles Sprague-Dawley (250 à 350 g) sont anesthésiés par une injection intra-péritonéale de pentobarbital sodique (60 mg/kg). La pression artérielle diastolique est enregistrée grâce à un cathéter (PE50) hépariné introduit dans la carotide gauche de l'animal, et relie à un calculateur de pression (Gould, Pressure Processor) par l'intermédiaire d'un capteur de pression Gould.

Un cathéter est introduit dans la jugulaire droite de l'animal pour l'injection des molécules à étudier.

L'animal est placé sous respiration assistée. Une section bilatérale des nerfs vagues est effectuée. Le rat est alors démédullé.

Après une période de stabilisation suffisante, l'étude de l'antagonisme des composés vis-à-vis de l'angiotensine II (Hypertensine Ciba) est abordée de la façon suivante.
1 - Trois injections consécutives d'angiotensine II (0,75 microgrammes/kg) espacées de 15 minutes permettent d'obtenir une réponse pressive reproductible et stable.
2 - Tout en gardant une périodicité de 15 minutes pour l'administration d'angiotensine II, les molécules à étudier sont injectées 5 minutes avant l'angiotensine II.

Les effets presseurs de l'angiotensine II en présence de l'antagoniste sont exprimés en pourcentage des effets presseurs de l'angiotensine II administrée seule. La dose inhibitrice à 50 % de l'effet étudié est ainsi déterminée

Chaque animal est considéré comme son propre témoin.

### Résultats :

| Produit de l'exemple | Dose inhibitrice à 50 % (mg/kg) (voie veineuse) |
|---|---|
| 3 | 0,08 |
| 4 | 0,09 |

## Revendications

1. Produits de formule (I) : 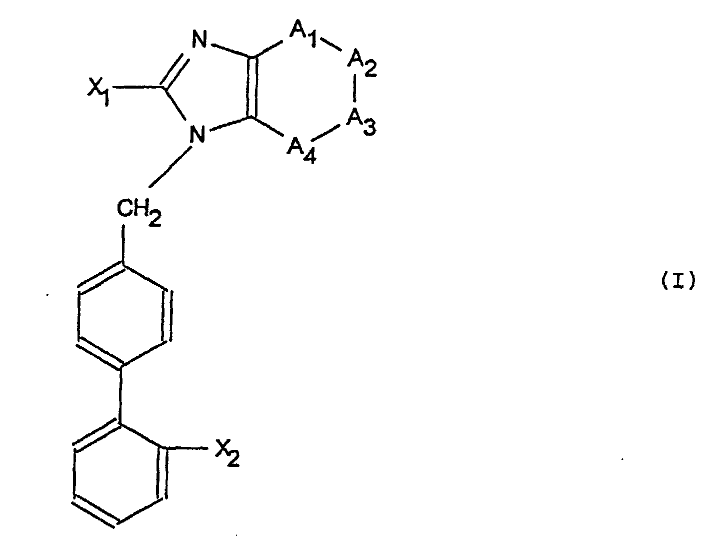
dans laquelle : X₁ représente un radical méthyle, éthyle, propyle, n-butyle, i-butyle ou terbutyle,
A₁ représente -S-, A₂ représente -CH₂,
A₃ représente -CH₂- ou -CH=, un atome d'hydrogène étant éventuellement substitué par un atome d'halogène, un radical formyle ou carboxy libre, salifié ou estérifié,
A₄ représente >C=O, >C=N-O-CH₂-COOH, >C=N-OH, >C=CH-COOH, 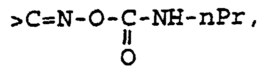 -CH₂- avec l'un ou les 2 atomes d'hydrogène substitués par un ou deux radicaux identiques ou différents choisis parmi les radicaux amino, hydroxyle et halogène, ou -CH= éventuellement substitué par un radical amino, hydroxyle ou un atome d'halogène
X₂ représente le radical tétrazolyle éventuellement salifié ou estérifié, le radical -SO₂-NH-CO-O-R₅, -SO₂-NH-CO-R₅,
-SO₂-N=CH-NR₆, ou -SO₂-NH-CO-NH-R₅ dans lesquels R₅ et R₆ identiques ou différents, sont choisis parmi l'atome d'hydrogène, le radical méthyle, éthyle et n-propyle,
lesdits produits de formule (I) étant sous toutes les formes isomères possibles racémiques, énantiomères et diastéréoisomères, ainsi que les sels d'addition avec les acides minéraux et organiques ou avec les bases minérales et organiques, desdits produits de formule (I).

2. Le produit suivant
acide 2-butyl 1- [[2'-[[(éthoxycarbonyl] amino] sulfonyl] (1,1'-biphényl) -4-yl] méthyl] 1,5,6,7-tétrahydrothiopyrano [2,3-d] imidazol 7-carboxylique, lesdits produits de formule (I) ce produit étant sous toutes les formes isomères possibles racémiques, énantiomères et diastéréoisomères, ainsi que les sels d'addition avec les acides minéraux et organiques ou avec les bases minérales et organiques, desdits produits de formule (I) de ce produit.

3. le 4'-[(2-butyl-7-oxo-1,5,6,7-tétrahydro-thiopyrano[2,3-d] imidazol-1-yl) méthyl] N-[(diméthylamino) méthylène) (1,1'-biphényl) 2-sulfonamide,
- le 4'-[(2-butyl-7-oxo-1,5,6,7-tétrahydro-thiopyrano[2,3-d] imidazol-1-yl) méthyl] (1,1'-biphényl) 2-sulfonamide,
- le (2-(4' -((2-butyl-1,5,6,7-tétrahydro-7-oxo-thiopyrano[2,3-d] imidazol-1-yl) méthyl) (1,1'-biphényl) sulfonyl) carbamate d'éthyle,
- le N-(2-(4' -((2-butyl-1,5,6,7-tétrahydro-7-oxo-thiopyrano[2,3-d] imidazol-1-yl) méthyl) (1,1'-biphényl) sulfonyl) N'-propyl-urée,
- le 4'-[(2-butyl-7-(hydroxyimino)-1,5,6,7-tétrahydro-thiopyrano[2,3-d] imidazol-1-yl) méthyl] (1,1'-biphényl) 2-sulfonamide,
- le 4'-[(2-butyl-7-((((propylamino) carbonyl) oxy) imino)-1,5,6,7-tétrahydro-thiopyrano[2,3-d] imidazol-1-yl) méthyl] N-[(propylamino) carbonyl] (1,1'-biphényl) 2-sulfonamide,
- 4'-[(2-butyl 7-(hydroxyimino) 1,5,6,7-tétrahydro thiopyrano[2,3-d]imidazol-1-yl) méthyl] N-((propylamino) carbonyl) (1,1'-biphényl) 2-sulfonamide,
- le 4'-[(2-butyl-1,7-dihydro-7-oxo-thiopyrano[2,3-d]imidazol-1-yl) méthyl] N-[(diméthylamino) méthylène) (1,1'-biphényl) 2-sulfonamide,
- le 4' -[(2-butyl-1,7-dihydro-7-oxo-thiopyrano[2,3-d]imidazol-1-yl) méthyl] (1,1'-biphényl) 2-sulfonamide,
- le 4'-[(2-butyl-1,7-dihydro-7-oxo-thiopyrano [2,3-d]imidazol-1-yl) méthyl] N-[(propylamino) carbonyl) (1,1'-biphényl) 2-sulfonamide.

4. Procédé de préparation des produits de formule (I) telle que définie à la revendication 1, **caractérisé en ce que** l'on fait réagir un composé de formule (II) : 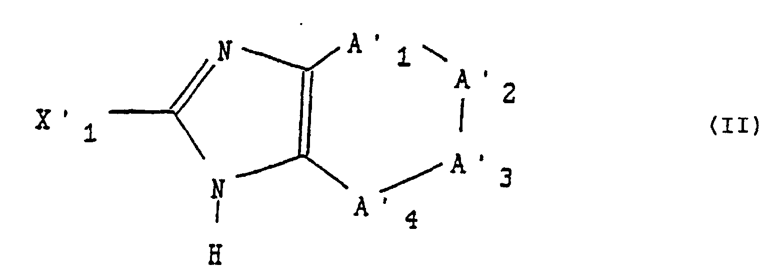 dans laquelle X'₁, A'₁, A'₂, A'₃ et A'₄ ont les significations indiquées à la revendication 1 respectivement pour X₁, A₁, A₂, A₃ et A₄, dans lesquelles les éventuelles fonctions réactives sont éventuellement protégées par des groupements protecteurs,
avec un composé de formule (III) : 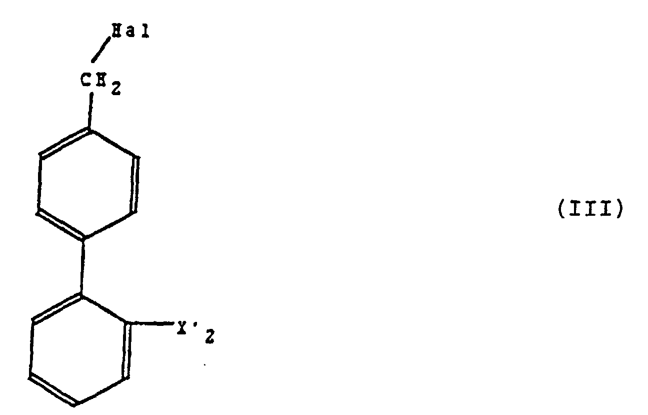 dans laquelle Hal représente un atome d'halogène, n₁ représente un entier de 0 à 4 et X'₂ a la signification indiquée à la revendication 1 pour X₂ dans laquelle les éventuelles fonctions réactives sont éventuellement protégées par des groupements protecteurs,
pour obtenir un produit de formule (IV) 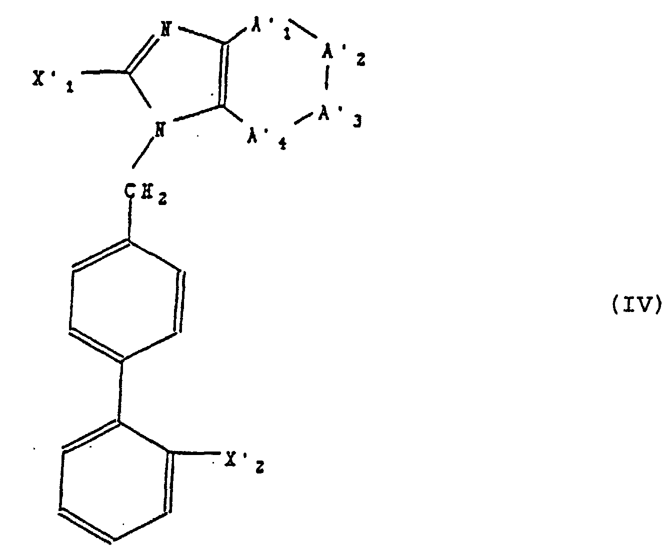 dans laquelle X'₁, A'₁, A'₂, A'₃, A'₄, X'₂ et n₁ ont les significations indiquées ci-dessus,
produit de formule (IV) pouvant déjà constituer un produit de formule (I), que le cas échéant, l'on transforme pour obtenir un autre produit de formule (I) ou produit de formule (IV) que l'on transforme en produit de formule (I) en soumettant ce produit de formule (IV) ou ce produit de formule (I), si désiré et si nécessaire, à l'une ou plusieurs des réactions suivantes, dans un ordre quelconque :
- élimination des groupements protecteurs que peuvent porter les fonctions réactives protégées,
- salification par un acide minéral ou organique ou par une base minérale ou organique pour obtenir le sel correspondant,
- estérification de fonction acide,
- saponification de fonction ester en fonction acide,
- transformation de fonction alkyloxy en fonction hydroxyle,
- transformation de la fonction cyano en fonction acide,
- réduction de la fonction carboxy en fonction alcool,
- oxydation de l'atome de soufre en sulfoxyde ou en sulfone,
- transformation de la fonction cétone en fonction oxime,
- transformation de la fonction cétone en fonction alcool,
- transformation de la fonction amine en fonction amide,
- substitution par un atome d'halogène,
- dédoublement des formes racémiques,
lesdits produits de formule (I) ainsi obtenus étant sous toutes les formes isomères possibles racémiques, énantiomères et diastéréoisomères.

5. Procédé de préparation du produit de formule (II) tel que défini à la revendication 4, **caractérisé en ce que** l'on fait réagir un composé de formule (Fa) : 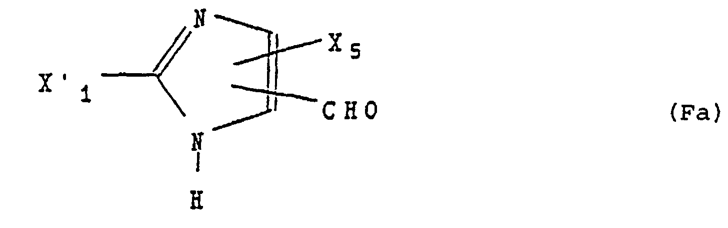 dans laquelle X₅ représente un radical alkyle, alcoxy, hydroxyle, formyle, alkylthio, phénylthio, benzylthio, carboxy libre, salifié ou estérifié ou amino, éventuellement substitués par un ou deux radicaux alkyke, tel que les radicaux alkyle et phényle de ces radicaux que peut comprendre ou représenter X₅ sont éventuellement substitués par un ou plusieurs radicaux choisis parmi les atomes d'halogène, les radicaux hydroxyle, alkyle, aralcoxy, et sont, si nécessaire, protégés,
avec un halogénure de lithium ou de magnésium de formule (Fb) :
A"₂-----A"₃-M Hal (Fb)
dans laquelle Hal représente un atome d'halogène. M représente un atome de lithium ou de magnésium et A"₂ A"₃ représente soit un radical éthényle non substitué ou substitué par 1, 2 ou 3 radicaux R'₁ et R'₂ tels que définis ci-dessus, dans lesquels les éventuelles fonctions réactives sont éventuellement protégées soit l'un de A"₂ et A"₃ représente un radical amino éventuellement substitué par un ou 2 radicaux R'₁ et R'₂ tels que définis ci-dessus, dans lesquels les éventuelles fonctions réactives sont éventuellement protégées ci-dessus, pour obtenir un produit de formule (Fc): (Fc) : 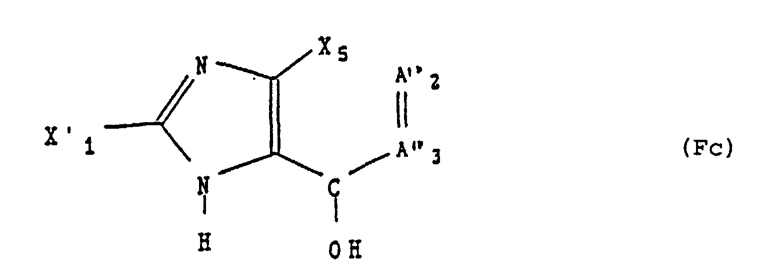 dans laquelle X'₁, X₅, A"₂ et A"₃ ont les significations indiquées ci-dessus, que l'on oxyde pour obtenir un composé de formule (Fd) : 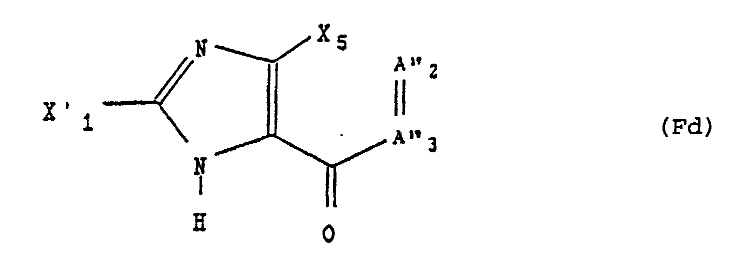 dans laquelle X'₁, X₅, A"₂ et A"₃ ont les significations indiquées ci-dessus, que l'on soumet à une réaction de cyclisation pour obtenir le produit de formule (Fe) : 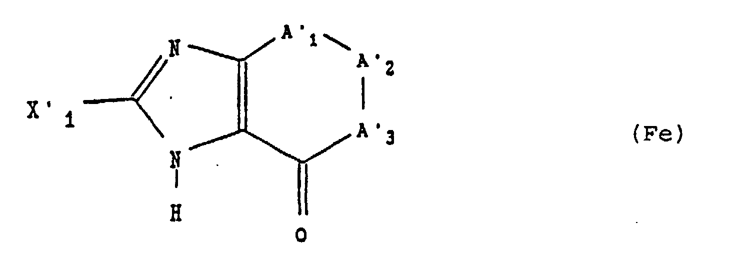 dans laquelle X'₁, A'₁, A'₂ et A'₃ ont les significations indiquées ci-dessus, produits de formule (Fe) pouvant constituer un produit de formule (II) que le cas échéant l'on transforme pour obtenir un autre produit de formule (II) ou produit de formule (Fe) que l'on transforme en produit de formule (II) en le soumettant, si désiré et si nécessaire, à l'une ou plusieurs des réactions suivantes, dans un ordre quelconque :
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction d'estérification de fonction acide,
- une réaction de saponification de fonction ester en fonction acide,
- une réaction de transformation de fonction alkyloxy en fonction hydroxyle,
- une réaction de transformation de la fonction cyano en fonction acide,
- une réaction de transformation de la fonction cétone en fonction cyano,
- une réaction de réduction de la fonction carboxy en fonction alcool,
- une réaction de dédoublement des formes racémiques, lesdits produits de formule (II) ainsi obtenus étant sous toutes les formes isomères possibles racémiques, énantiomères et diastéréoisomères.

6. A titre de médicaments, les produits tels que définis par la formule (I) de la revendication 1, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

7. Les compositions pharmaceutiques contenant à titre de principe actif, l'un au moins des médicaments tels que définis à la revendication 6.

8. A titre de produits industriels nouveaux, les composés de formule (II).

## Claims

1. Products of formula (I): 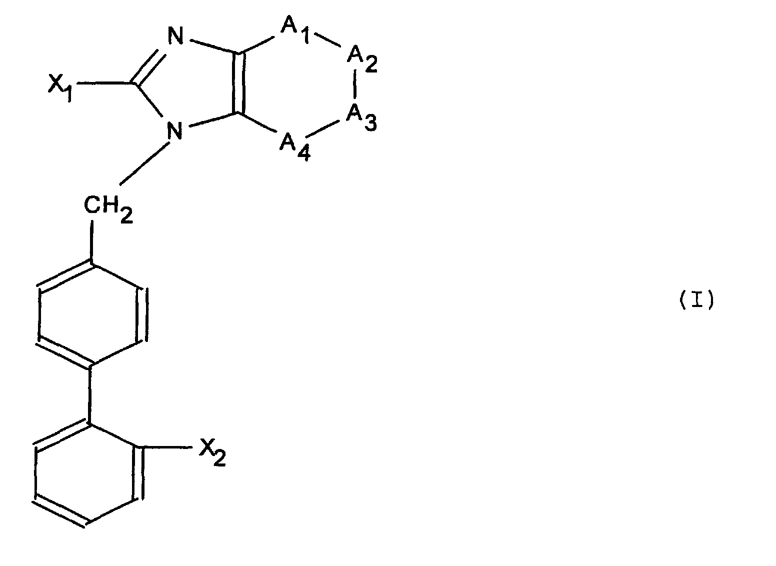
in which: X₁ represents a methyl, ethyl, propyl, n-butyl, i-butyl or terbutyl radical,
A₁ represents -S-, A₂ represents -CH₂,
A₃ represents -CH₂- or -CH=, a hydrogen atom being optionally substituted by a halogen atom, a formyl or free, salified or esterified carboxy radical,
A₄ represents >C=O, >C=N-O-CH₂-COOH, >C=N-OH, >C=CH-COOH, 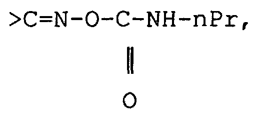 -CH₂- with one or both hydrogen atoms substituted by one or two identical or different radicals chosen from the amino, hydroxyl and halogen radicals, or -CH= optionally substituted by an amino, hydroxyl radical or a halogen atom
X₂ represents the optionally salified or esterified tetrazolyl radical, the -SO₂-NH-CO-O-R₅, -SO₂-NH-CO-R₅, -SO₂-N=CH-NR₆, or
-SO₂-NH-CO-NH-R₅ radical in which R₅ and R₆ identical or different, are chosen from the hydrogen atom, the methyl, ethyl and n-propyl radical,
said products of formula (I) being in all racemic, enantiomeric and diastereoisomeric isomer forms, as well as the addition salts with mineral and organic acids or with mineral and organic bases of said products of formula (I).

2. The following product
2-butyl 1-[[2'- [[(ethoxycarbonyl] amino] sulphonyl] (1,1'-biphenyl)-4-yl] methyl] 1,5,6,7-tetrahydrothiopyrano [2,3-d] imidazol 7-carboxylic acid, said products of formula (I), this product, being in all possible racemic, enantiomeric and diastereoisomeric isomer forms, as well as the addition salts with mineral and organic acids or with mineral and organic bases of said products of formula (I) of this product.

3. 4'-[(2-butyl-7-oxo-1,5,6,7-tetrahydro-thiopyrano[2,3-d] imidazol-1-yl) methyl] N-[(dimethylamino) methylene) (1,1'-biphenyl) 2-sulphonamide,
- 4'-[(2-butyl-7-oxo-1,5,6,7-tetrahydro-thiopyrano[2,3-d] imidazol-1-yl) methyl] (1,1'-biphenyl) 2-sulphonamide,
- ethyl (2-(4'-((2-butyl-1,5,6,7-tetrahydro-7-oxothiopyrano[2,3-d] imidazol-1-yl) methyl) (1,1'-biphenyl) sulphonyl) carbamate,
- N-(2-(4'-((2-butyl-1,5,6,7-tetrahydro-7-oxothiopyrano[2,3-d] imidazol-1-yl) methyl) (1,1'-biphenyl) sulphonyl) N'-propyl-urea,
- 4'-[(2-butyl-7-(hydroxyimino )-1,5,6,7-tetrahydrothiopyrano[2,3-d] imidazol-1-yl) methyl] (1,1'-biphenyl) 2-sulphonamide,
- 4'-[(2-butyl-7-((((propylamino) carbonyl) oxy) imino)-1,5,6,7-tetrahydro-thiopyrano[2,3-d] imidazol-1-yl) methyl] N-[(propylamino) carbonyl] (1,1'-biphenyl) 2-sulphonamide,
- 4'-[(2-butyl 7-(hydroxyimino) 1,5,6,7-tetrahydro thiopyrano[2,3-d]imidazol-1-yl) methyl] N-((propylamino) carbonyl) (1,1'-biphenyl) 2-sulphonamide,
- 4'-[(2-butyl-1,7-dihydro-7-oxo-thiopyrano[2,3-d]imidazol-1-yl) methyl] N-[(dimethylamino) methylene) (1,1'-biphenyl) 2-sulphonamide,
- 4'-[(2-butyl-1,7-dihydro-7-oxo-thiopyrano[2,3-d]imidazol-1-yl) methyl] (1,1'-biphenyl) 2-sulphonamide,
- 4'-[(2-butyl-1,7-dihydro-7-oxo-thiopyrano[2,3-d]imidazol-1-yl) methyl] N-[(propylamino) carbonyl) (1,1'-biphenyl) 2-sulphonamide.

4. Process for the preparation of the products of formula (I) as defined in claim 1, **characterized in that** a compound of formula (II): 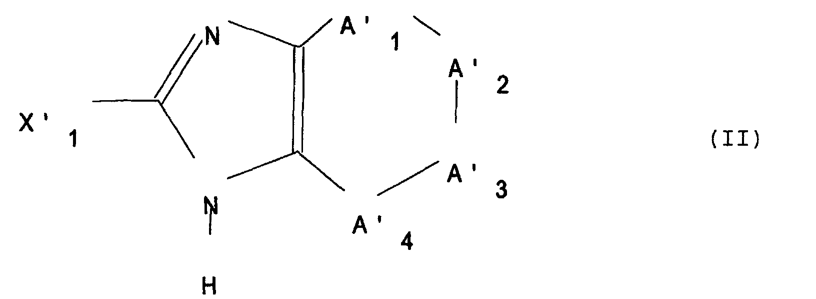 in which X'₁, A'₁, A'₂, A'₃ and A'₄ have the meanings indicated in claim 1 for X₁, A₁, A₂, A₃ and A₄ respectively, in which the optional reactive functions are optionally protected by protective groups,
is reacted with a compound of formula (III): 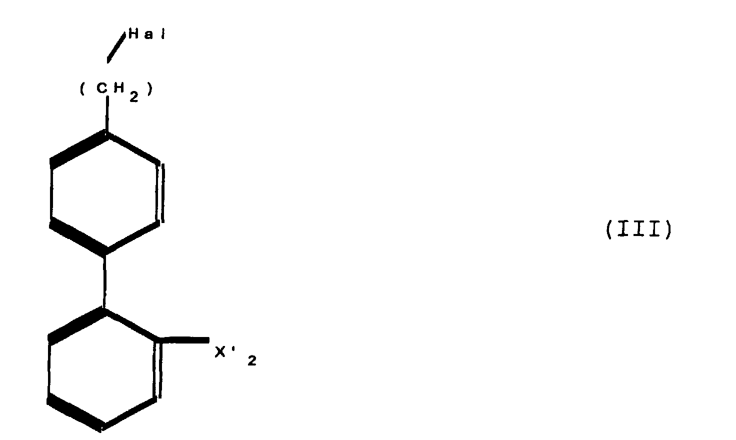 in which Hal represents a halogen atom, n₁ represents an integer from 0 to 4 and X'₂ has the meaning indicated in claim 1 for X₂ in which the optional reactive functions are optionally protected by protective groups,
in order to obtain a product of formula (IV): 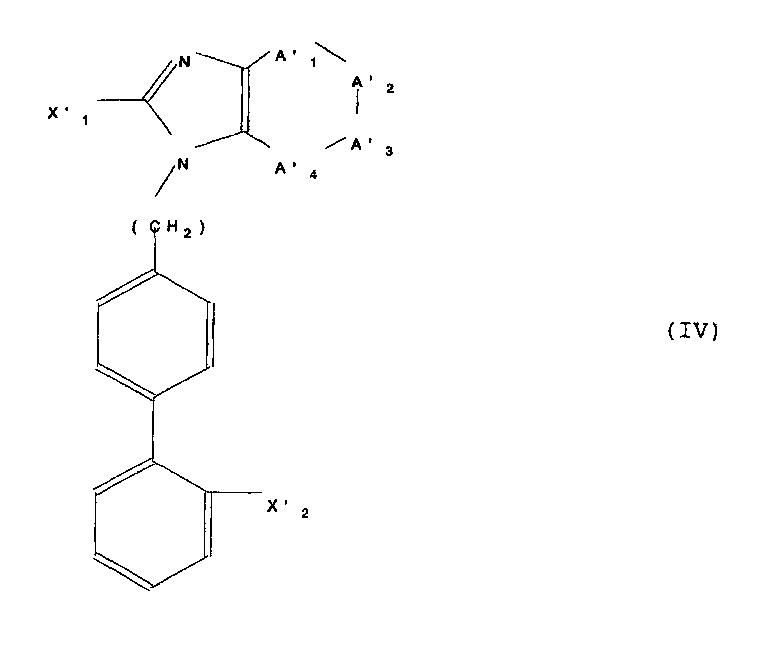 in which X'₁, A'₁, A'₂, A'₃, A'₄, X'₂ and n₁ have the meanings indicated above,
product of formula (IV) already able to constitute a product of formula (I), if appropriate, is converted in order to obtain another product of formula (I) or product of formula (IV) which is converted to a product of formula (I) by subjecting this product of formula (IV) or this product of formula (I), if desired and if necessary, to one or more of the following reactions, in any order:
- elimination of the protective groups which can be carried by the protected reactive functions,
- salification by a mineral or organic acid or by a mineral or organic base in order to obtain the corresponding salt,
- esterification of the acid function,
- saponification of the ester function to an acid function,
- conversion of the alkyloxy function to a hydroxyl function,
- conversion of the cyano function to an acid function,
- reduction of the carboxy function to an alcohol function,
- oxidation of the sulphur atom to sulphoxide or to sulphone,
- conversion of the ketone function to an oxime function,
- conversion of the ketone function to an alcohol function,
- conversion of the amine function to an amide function,
- substitution by a halogen atom,
- resolving the racemic forms, said products of formula (I) obtained in this way being in all possible racemic, enantiomeric and diastereoisomeric isomer forms.

5. Process for the preparation of the product of formula (II) as defined in claim 4, **characterized in that** a compound of formula (Fa): 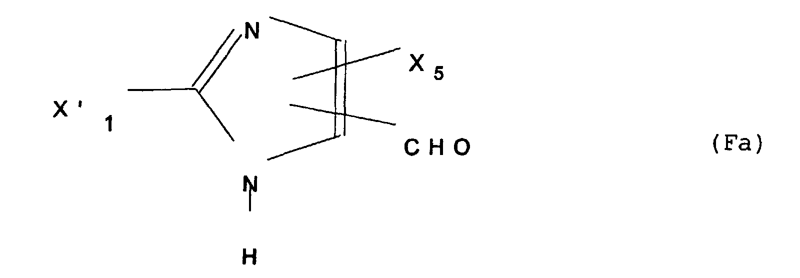 in which X₅ represents an alkyl, alkoxy, hydroxyl, formyl, alkylthio, phenylthio, benzylthio, free, salified or esterified carboxy or amino radical, optionally substituted by one or two alkyl radicals, such that the alkyl and phenyl radicals of these radicals which can comprise or be represented by X₅ are optionally substituted by one or more radicals chosen from the halogen atoms, the hydroxyl, alkyl, aralkoxy radicals, and are, if necessary, protected, is reacted with a lithium or magnesium halide of formula (Fb) :
A"₂ ---- A"₃-M Hal (Fb)
in which Hal represents a halogen atom. M represents a lithium or magnesium atom and A"₂ A"₃ represent either an ethenyl radical non-substituted or substituted by 1, 2 or 3 R'₁ and R'₂ radicals as defined above, in which the optional reactive functions are optionally protected or one of A"₂ and A"₃ represents an amino radical optionally substituted by one or two R'₁ and R'₂ radicals as defined above, in which the optional reactive functions are optionally protected above, in order to obtain a product of formula (Fc):
(Fc): 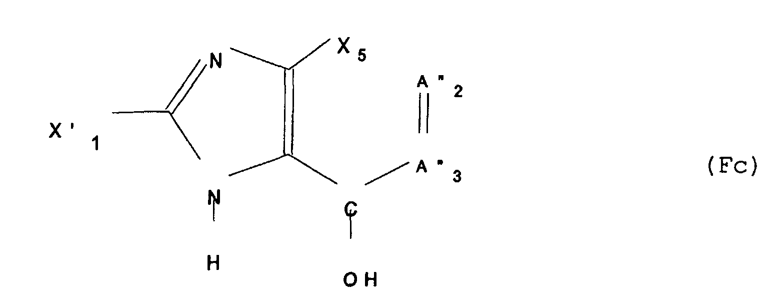 in which X'₁, X₅, A"₂ and A"₃ have the meanings indicated above, which is oxidized in order to obtain a compound of formula (Fd): 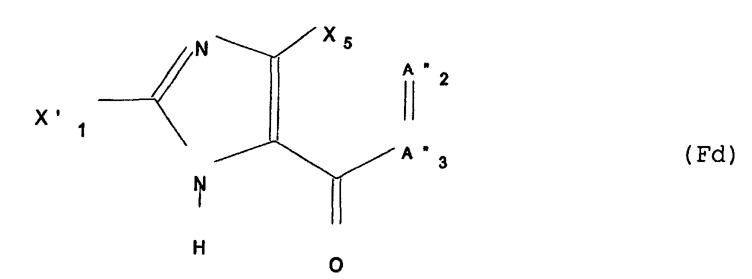 in which X'₁, X₅, A"₂ and A"₃ have the meanings indicated above, which is subjected to a cyclization reaction in order to obtain the product of formula (Fe): 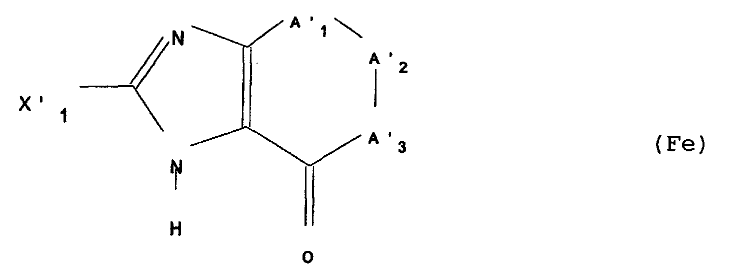 in which X'₁, A'₁, A'₂ and A'₃ have the meanings indicated above, which products of formula (Fe) able constitute a product of formula (II) which if appropriate is converted in order to obtain another product of formula (II) or product of formula (Fe) which is converted to a product of formula (II) by subjecting it, if desired and if necessary, to one or more of the following reactions, in any order:
- an elimination reaction of the protective groups which can be carried by the protected reactive functions,
- a salification reaction by a mineral or organic acid or by a mineral or organic base in order to obtain the corresponding salt,
- an esterification reaction of the acid function,
- a saponification reaction of the ester function to an acid function,
- a conversion reaction of the alkyloxy function to a hydroxyl function,
- a conversion reaction of the cyano function to an acid function,
- a conversion reaction of the ketone function to a cyano function,
- a reduction reaction of the carboxy function to an alcohol function,
- a resolution reaction of the racemic forms, said products of formula (II) obtained in this way being in all possible racemic, enantiomeric and diastereoisomeric isomer forms.

6. As medicaments, the products as defined by formula (I) of claim 1, said products of formula (I) being in all possible racemic, enantiomeric and diastereoisomeric isomer forms, as well as the addition salts with pharmaceutically acceptable mineral and organic acids or with mineral and organic bases of said products of formula (I).

7. The pharmaceutical compositions containing as active ingredient, at least one of the medicaments as defined in claim 6.

8. As new industrial products, the compounds of formula (II).

## Patentansprüche

1. Produkte der Formel (I) 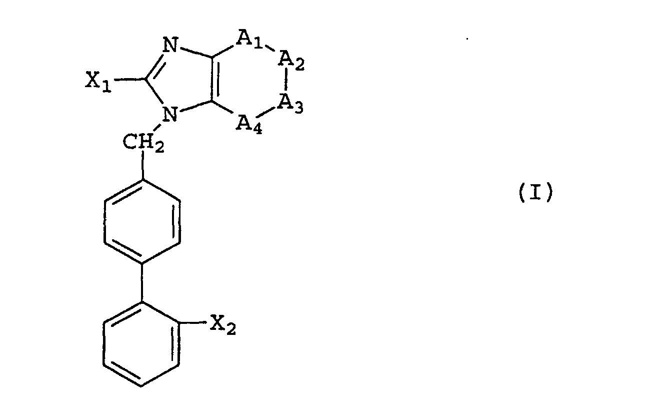 in der
X₁ einen Rest Methyl, Ethyl, Propyl, n-Butyl, i-Butyl oder tert.-Butyl darstellt,
A₁ -S- darstellt, A₂ -CH₂- darstellt,
A₃ -CH₂- oder -CH= darstellt, wobei ein Wasserstoffatom gegebenenfalls substituiert ist durch ein Halogenatom, einen Rest Formyl oder Carboxy, frei, in Salz überführt oder verestert,
A₄ >C=O, >C=N-O-CH₂-COOH, >C=N-OH, >C=CH-COOH, 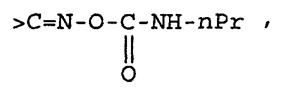 -CH₂-, worin eines oder beide Wasserstoffatome substituiert sind durch ein oder zwei gleiche oder verschiedene Reste, ausgewählt unter den Resten Amino, Hydroxyl und Halogen, oder -CH= darstellt, gegebenenfalls substituiert durch einen Rest Amino, Hydroxyl oder ein Halogenatom,
X₂ den Rest Tetrazolyl, gegebenenfalls in Salz überführt oder verestert, den Rest -SO₂-NH-CO-O-R₅, -SO₂-NH-CO-R₅, -SO₂-N=CH-NR₆ oder
-SO₂-NH-CO-NH-R₅ bedeutet, worin R₅ und R₆, gleich oder verschieden, ausgewählt werden unter dem Wasserstoffatom, dem Rest Methyl, Ethyl, n-Propyl,
wobei die genannten Produkte der Formel (I) in allen möglichen isomeren racemischen Formen, Enantiomeren und Diastereoisomeren vorliegen können, sowie die Additionssalze der genannten Produkte der Formel (I) mit Mineralsäuren und organischen Säuren oder mit Mineralbasen und organischen Basen.

2. Das folgende Produkt:
2-Butyl-1-{{2'-[[(ethoxycarbonyl)-amino]-sulfonyl]-(1,1'-biphenyl)-4-yl}-methyl}-1,5,6,7-tetrahydrothiopyrano[2,3-d]-imidazol-7-carbonsäure, wobei dieses Produkt von den genannten Produkten der Formel (I) in allen möglichen isomeren racemischen Formen, Enantiomeren und Diastereoisomeren vorliegen kann, sowie die Additionssalze dieses Produktes von den genannten Produkten der Formel (I) mit Mineralsäuren und organischen Säuren oder mit Mineralbasen und organischen Basen.

3. 4'-[(2-Butyl-7-oxo-1,5,6,7-tetrahydro-thiopyrano[2,3-d]-imidazol-1-yl)-methyl]-N-[(dimethylamino)-methylen]-(1,1'-biphenyl)-2-sulfonamid,
- 4'-[(2-Butyl-7-oxo-1,5,6,7-tetrahydro-thiopyrano[2,3-d]-imidazol-1-yl)-methyl]-(1,1'-biphenyl)-2-sulfonamid,
- {2-(4'-(2-Butyl-7-oxo-1,5,6,7-tetrahydro-thiopyrano[2,3-d]-imidazol-1-yl)-methyl]-(1,1'-biphenyl)-sulfonyl}-ethylcarbamat,
- N-{2-[4'-(2-Butyl-7-oxo-1,5,6,7-tetrahydro-thiopyrano[2,3-d]-imidazol-1-yl)-methyl ]-(1,1'-biphenyl)-sulfonyl}-N'-propyl-harnstoff,
- 4'-{[2-Butyl-7-(hydroxyimino)-1,5,6,7-tetrahydro-thiopyrano-[2,3-d]-imidazol-1-yl]-methyl}-(1,1'-biphenyl)-2-sulfonamid,
- 4'-{{2-Butyl-7-[[((propylamino)-carbonyl)-oxy]-imino]-1,5,6,7-tetrahydro-thiopyrano[2,3-d]-imidazol-1-yl}-methyl}-N-[(propylamino)-carbonyl]-(1,1'-biphenyl)-2-sulfonamid,
- 4'-{[2-Butyl-7-(hydroxyimino)-1,5,6,7-tetrahydro-thiopyrano-[2,3-d]-imidazol-1-y1]-methyl}-N-[(propylamino)-carbonyl]-(1,1'biphenyl)-2-sulfonamid,
- 4'-[(2-Butyl-1,7-dihydro-7-oxo-thiopyrano -[2,3-d]-imidazol-1-yl)-methyl]-N -[(dimethylamino)-methylen ]-(1,1'-biphenyl)-2-sulfonamid,
- 4'-[(2-Butyl-1,7-dihydro-7-oxo-thiopyrano-[2,3-d]-imidazol-1-yl)-methyl]-(1,1'-biphenyl)-2-sulfonamid,
- 4'- [(2-Butyl-1,7-dihydro-7-oxo-thiopyrano- [2,3-d] -imidazol-1-yl)-methyl]-N-[(propylamino)-carbonyl]-(1,1'-biphenyl)-2-sulfonamid.

4. Verfahren zur Herstellung von Produkten der Formel (I) wie in Anspruch 1 definiert, **dadurch gekennzeichnet, daß** man eine Verbindung der Formel (II) 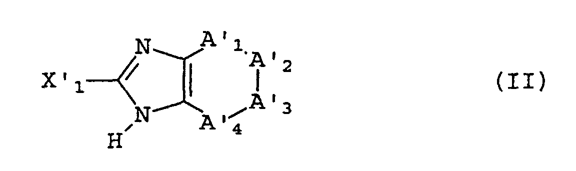 in der X'₁, A'₁, A'₂, A'₃ und A'₄ die in Anspruch 1 jeweils für X₁, A₁, A₂, A₃ und A₄ angegebenen Bedeutungen besitzen, bei denen die eventuellen reaktiven Funktionen gegebenenfalls durch Schutzgruppen geschützt sind, mit einer Verbindung der Formel (III) 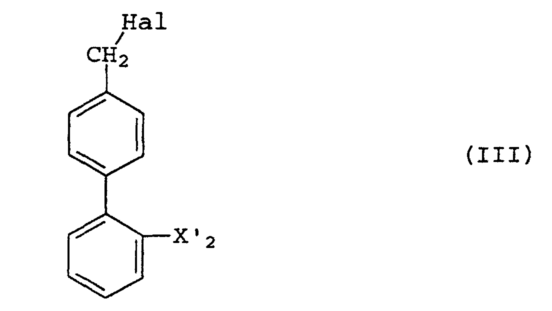 zur Reaktion bringt, in der Hal ein Halogenatom ist, n₁ eine ganze Zahl von 0 bis 4 darstellt und X'₂ die in Anspruch 1 für X₂ angegebene Bedeutung besitzt, wobei die eventuellen reaktiven Funktionen gegebenenfalls durch Schutzgruppen geschützt sind, um ein Produkt der Formel (IV) 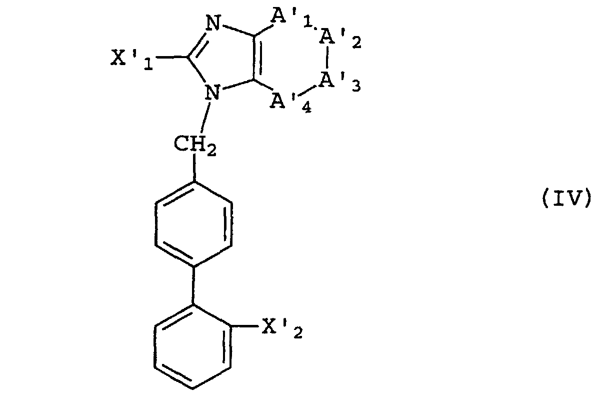 zu erhalten, in der X'₁, A'₁, A'₂, A'₃, A'₄, X'₂ und n₁ die oben angegebenen Bedeutungen besitzen,
wobei man gegebenenfalls das Produkt der Formel (IV), das bereits ein Produkt der Formel (I) darstellen kann, gegebenenfalls umwandelt, um ein anderes Produkt der Formel (I) oder Produkt der Formel (IV) zu erhalten, das man in das Produkt der Formel (I) umwandelt, indem man dieses Produkt der Formel (IV) oder dieses Produkt der Formel (I), wenn erwünscht und wenn erforderlich, einer oder mehreren der folgenden Reaktionen in irgendeiner Reihenfolge unterzieht:
- Entfernung der Schutzgruppen, die von den geschützten reaktiven Funktionen getragen werden können,
- Salzbildung durch eine Mineralsäure oder organische Säure oder durch eine Mineralbase oder organische Base, um das entsprechende Salz zu erhalten,
- Veresterung der Säurefunktion,
- Verseifung der Esterfunktion zur Säurefunktion,
- Umwandlung der Alkyloxyfunktion zur Hydroxylfunktion,
- Umwandlung der Cyanofunktion zur Säurefunktion,
- Reduktion der Carboxyfunktion zur Alkoholfunktion,
- Oxidation des Schwefelatoms zum Sulfoxid oder zum Sulfon,
- Umwandlung der Ketonfunktion zur Oximfunktion,
- Umwandlung der Ketonfunktion zur Alkoholfunktion,
- Umwandlung der Aminfunktion zur Amidfunktion,
- Substitution durch ein Halogenatom,
- Aufspaltung der racemischen Formen,
wobei die genannten Produkte der Formel (I), die auf diese Weise erhalten wurden, in allen möglichen isomeren racemischen Formen, Enantiomeren und Diastereoisomeren vorliegen können.

5. Verfahren zur Herstellung des Produktes der Formel (II) wie in Anspruch 4 definiert, **dadurch gekennzeichnet, daß** man eine Verbindung der Formel (Fa) 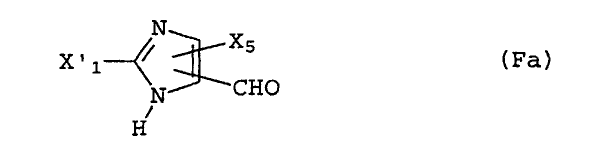 in der X₅ einen Rest Alkyl, Alkoxy, Hydroxyl, Formyl, Alkylthio, Phenylthio, Benzylthio, Carboxy, frei, in Salz überführt oder verestert, oder Amino darstellt, gegebenenfalls substituiert durch einen oder zwei Reste Alkyl, wie die Reste Alkyl und Phenyl, wobei diese Reste, die von X₅ umfaßt oder dargestellt werden können, gegebenenfalls substituiert sind durch einen oder mehrere Reste, ausgewählt unter den Halogenatomen, den Resten Hydroxyl, Alkyl, Aralkoxy, die wenn erforderlich, geschützt sind,
mit einem Lithiumhalogenid oder Magnesiumhalogenid der Formel (Fb)
A"₂ ----- A"₃―M Hal (Fb)
zur Reaktion bringt, in der Hal ein Halogenatom ist, M ein Lithiumatom oder Magnesiumatom darstellt und A"₂, A"₃ entweder einen Rest Ethenyl bedeuten, nicht substituiert oder substituiert durch 1, 2 oder 3 wie oben definierte Reste R'₁ und R'₂, worin die eventuellen reaktiven Funktionen gegebenenfalls geschützt sind, oder eines von A"₂ und A"₃ stellt einen Rest Amino dar, gegebenenfalls substituiert durch einen oder zwei wie oben definierte Reste R'₁ und R'₂, worin die eventuellen reaktiven Funktionen gegebenenfalls wie oben geschützt sind, um ein Produkt der Formel (Fc) 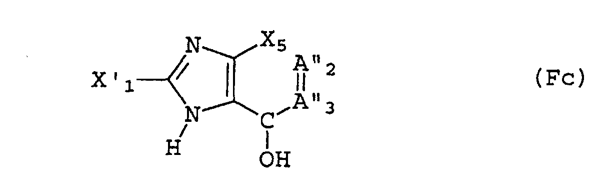 zu erhalten, in der X'₁, X₅, A"₂ und A"₃ die oben angegebenen Bedeutungen besitzen, das man oxidiert, um eine Verbindung der Formel (Fd) 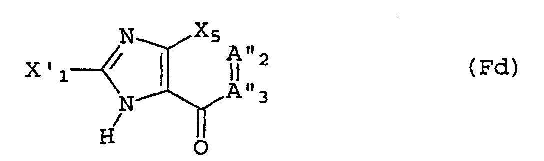 zu erhalten, in der X'₁, X₅, A"₂ und A"₃ die oben angegebenen Bedeutungen besitzen, das man einer Reaktion zur Cyclisierung unterzieht, um das Produkt der Formel (Fe) 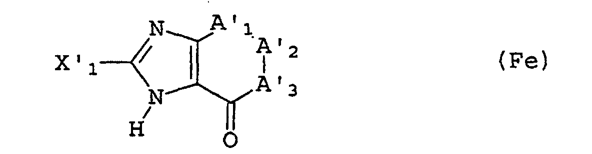 zu erhalten, in der X'₁, A'₁, A'₂ und A'₃ die oben angegebenen Bedeutungen besitzen, wobei die Produkte der Formel (Fe) ein Produkt der Formel (II) bilden können, das man gegebenenfalls umwandelt, um ein anderes Produkt der Formel (II) oder Produkt der Formel (Fe) zu erhalten, das man in das Produkt der Formel (II) umwandelt, indem man es, wenn erwünscht und wenn erforderlich, einer oder mehreren der folgenden Reaktionen in irgendeiner Reihenfolge unterzieht:
- einer Reaktion zur Entfernung der Schutzgruppen, die von den geschützten reaktiven Funktionen getragen werden können,
- einer Reaktion zur Salzbildung durch eine Mineralsäure oder organische Säure oder durch eine Mineralbase oder organische Base, um das entsprechende Salz zu erhalten,
- einer Reaktion zur Veresterung der Säurefunktion,
- einer Reaktion zur Verseifung der Esterfunktion zur Säurefunktion,
- einer Reaktion zur Umwandlung der Alkyloxyfunktion zur Hydroxylfunktion,
- einer Reaktion zur Umwandlung der Cyanofunktion zur Säurefunktion,
- einer Reaktion zur Umwandlung der Ketonfunktion zur Cyanofunktion,
- einer Reaktion zur Reduktion der Carboxyfunktion zur Alkoholfunktion,
- einer Reaktion zur Aufspaltung der racemischen Formen,
wobei die genannten Produkte der Formel (II), die auf diese Weise erhalten wurden, in allen möglichen isomeren racemischen Formen, Enantiomeren und Diastereoisomeren vorliegen können.

6. Als Arzneimittel die Produkte wie durch die Formel (I) von Anspruch 1 definiert, wobei die genannten Produkte der Formel (I) in allen möglichen isomeren racemischen Formen, Enantiomeren und Diastereoisomeren vorliegen können, sowie die Additionssalze der genannten Produkte der Formel (I) mit pharmazeutisch akzeptablen Mineralsäuren und organischen Säuren oder mit Mineralbasen und organischen Basen.

7. Pharmazeutische Zusammensetzungen, enthaltend als Wirkstoff mindestens eines der Arzneimittel wie in Anspruch 6 definiert.

8. Als neue industrielle Produkte die Verbindungen der Formel (II) .
